# EUROPEAN PATENT APPLICATION

(11) **EP 2 397 098 A1**
(43) Date of publication of application: **21.12.2011**
(21) Application number: 10741100.1
(22) Date of filing: 12.02.2010
(51) Int. Cl.: A61B 19/00, A61B 17/16, B23B 39/14

(54) **NAVIGATION SYSTEM FOR REMOTE-CONTROLLED ACTUATOR**

(30) Priority: 16.02.2009 JP 2009032882; 17.02.2009 JP 2009033667
(71) Applicant: NTN Corporation, Osaka-shi, Osaka 550-0003 (JP)
(72) Inventor: NISHIO, Yukihiro, Iwata-shi Shizuoka 438-0037 (JP); NAGANO, Yoshitaka, Iwata-shi Shizuoka 438-0037 (JP)
(74) Representative: Hirsch & Associés
(86) International application number: PCT/JP2010/000867
(87) International publication number: WO 2010/092820

(57) **Abstract**

A remote controlled actuator (5) includes a distal end member (2) fitted to a distal end of a spindle guide section (3) for alteration in attitude, and a tool (1) rotatably supported by the distal end member. The navigation system includes a tool and tool marker relative position storage section (54) for storing the relative position of a processing member of the tool relative to the tool marker, and a relative relation storage section (55) having recorded therein the relative position and attitude of the tool marker relative to a main body marker (7A) for each angle of rotation of the distal end member, and a tool processing member position estimator (56) estimates the position of the processing member of the tool from the attitude and position of the main body marker during the actual procedure and the angle of rotation of the distal end member.

## Description

### CROSS REFERENCE TO THE RELATED APPLICATION

This application is based on and claims Convention priority to Japanese patent applications No. 2009-032882, filed February 16, 2009, and No. 2009-033667, filed February 17, 2009, the entire disclosures of which are herein incorporated by reference as a part of this application.

### BACKGROUND OF THE INVENTION

### (Field of the Invention)

The present invention relates to a navigation system for a remote controlled actuator of a kind, which is used in medical and mechanical processing applications and capable of altering the attitude of a machine tool by remote control.

### (Description of Related Art)

Remote controlled actuators are currently available; some are used in the medical field for osteo treatment and some are used in the mechanical processing field for drilling and cutting a bone. Any of those remote controlled actuators controls by remote control a machine tool fitted to a distal end of an elongated pipe of a linear or curved configuration. However, since the conventional remote controlled actuator is designed solely to control only the rotation of the machine tool by remote control, difficulties have been encountered in processing of a complicated shape and processing at a site difficult to view with eyes from the outside in the medical field. Also, in the drilling process, the capability of processing not only the linear line, but also the curved configuration is often required. In addition, in the cutting process, the capability is required to perform the process at a site deep in grooves. In the following description, conventional art and problems inherent in the remote controlled actuator will be discussed with reference to the medical field.

In the orthopedic field, the artificial joint replacement is well known, in which a joint, of which bone has been abraded by due to bone deterioration, is replaced with an artificial joint. The joint replacement surgery requires a living bone of a patient to be processed to enable an artificial joint to be implanted. In order to enhance the strength of postoperative adhesion between the living bone and the artificial joint, such processing is required to be performed precisely and accurately in conformity to the shape of the artificial joint.

By way of example, during the hip join replacement surgery, a thigh bone is opened to secure access of an artificial joint into the femoral marrow cavity. In order to secure a strength of contact between the artificial joint and the bone, surfaces of contact of the artificial joint and the bore must be large and so the opening for insertion of the artificial joint is processed to represent an elongated shape extending deep into the bone. As a medical actuator used in cutting the bone in a manner described above, the actuator is known, in which a tool is rotatably provided in a distal end of an elongated pipe and, on the other hand, a drive source such as, for example, a motor is mounted on a proximal end of the pipe so that the tool can be driven through a rotary shaft disposed inside the elongated pipe. (See, for example, the Patent Document 1 listed below.) Since in this type of medical actuator a rotatable element that is exposed bare to the outside is only the tool at the distal end of the elongated pipe, the tool can be inserted deep into the bone.

The surgical operation for artificial joint replacement generally accompanies skin incision and muscular scission. In other words, the human body must be invaded. In order to minimize the postoperative trace, it is quite often desirable that the elongated pipe referred to above is not necessarily straight, but is moderately curved. To meet with this desire, the following technique has hitherto been suggested. For example, the Patent Document 2 listed below discloses the elongated pipe having its intermediate portion curved double to displace an axial position of the distal end of the pipe relative to the longitudinal axis of the proximal end of the same pipe. To make the axial position of the distal end of the pipe relative to the longitudinal axis of the proximal end of the same pipe is also known from other publications. Also, the Patent Document 3 listed below discloses the elongated pipe rotated 180°.

If in a condition, in which the artificial joint is inserted into an artificial joint insertion hole formed in the living bone, a large gap exist between the living bone and the artificial joint, a large length of time is required to accomplish the postoperative adhesion between the living bone and the artificial joint and, therefore, it is considered desirable that the gap should be as small as possible. Also, it is important that respective surfaces of contact between the living bone and the artificial joint be smooth, and accordingly, a high precision is required in processing the artificial joint insertion hole. Whatever the pipe take any shape, the working range of the tool is limited by the shape of the pipe and, therefore, it is difficult to widen the working range of the tool to process the artificial joint insertion hole so that the living bone and the artificial joint may can have smooth contact surfaces and, yet, the gap between the living bone and the artificial joint may be small while skin incision and muscular scission are minimized at the same time.

In general, it is quite often that the patient's bone, where an artificial joint is to be implanted, exhibits a strength lowered as a result of aging and, in a certain case, the bone itself is deformed. Accordingly, the processing of the artificial joint insertion hole is more difficult to achieve than generally considered.

In view of the foregoing, the applicant or assignee of the present invention has attempted to provide a remote control actuator of a type designed to enable the processing of the artificial joint insertion hole to be relatively easily and accurately accomplished. For this purpose the remote controlled actuator of the type referred to above includes an elongated spindle guide section of a pipe-like contour, provided in an actuator main body, and a distal end member provided at a distal end portion of the spindle guide section for rotatably supporting a processing tool in a fashion capable of being altered in attitude so that the distal end member can be altered in attitude by remote control. This is because if the attitude of the tool can be changed, the tool can be maintained at a proper attitude regardless of the shape of the pipe. It is eventually pointed out that although the medical actuator of a type having no elongated pipe such as, for example, the spindle guide section, in which a portion provided with a processing tool is changeable in attitude relative to the grip that is held by hand, is currently available in the medical field (See, for example, the Patent Document 4 listed below.), nothing has yet been suggested which has a capability of changing the attitude of the tool by remote control.

Where the artificial joint insertion hole is to be processed in the bone with the use of the remote controlled actuator, it is quite often that the tool cannot be directly viewed with eyes and, therefore, a navigation system is needed in order to grasp the position of the tool. For this type of the navigation system, the following techniques have hitherto been well known in the art.

The Patent Document 5 listed below discloses the navigation system, in which a marker is applied to the bone so that the position of the bone can be measured by the detection of the marker with the use of an optical sensor. Using this technique, when a marker is applied not only to the bone, but also to the body of a remote controlled actuator, which is a stationary portion of such actuator in such case, respective positions of the bone and the actuator main body can be measured.

The Patent Document 6 also listed below discloses the navigation system, in which a marker, which is formed in a specific pattern, not in a dot, is applied to the actuator main body so that both of the position of the marker and the attitude of the actuator main body to which the marker has been applied can be detected by the detection of the pattern of that marker with the use of a marker detecting machine. Once the attitude of the actuator main body is determined, the position of the tool can be estimated from the relative positional relation between the distal end member and the tool and the site of the actuator main body where the marker has been applied. It is, however, to be noted that the relative positional relation between the distal end member and the tool and the site of the actuator main body where the marker has been applied is measured beforehand and is recorded and stored as a positional relational information.

It occurs quite often that the tool is replaced with a different type thereof depending on an object to be processed and/or upon, for example, wear, and, therefore, the positional relational information to be used in estimation of the position of the tool is needed to be updated each time the replacement takes place. As such the Patent Document 6 discloses the use of a switch operatively linked with removal of the tool to provide a piece of information with which the operator of the remote controlled actuator can be informed of the necessity of updating of the positional relational information.

The Patent Document 7 listed below discloses the navigation system, in which a second marker different from a first marker fitted to the actuator main body is prepared for use and in which the tool is brought into contact with the second marker and, using a positional relation between the actuator main body and the second marker then measured, the position of the tool is estimated on the basis of the positional relation between the actuator main body and the first marker measured during the procedure.

### [Prior Art Literature]

[Patent Document 1] JP Laid-open Patent Publication No. 2007-301149
[Patent Document 2] United State Patent No. 4,466,429
[Patent Document 3] United State Patent No. 4,265,231
[Patent Document 4] JP Laid-open Patent Publication No. 2001-17446
[Patent Document 5] United State Patent No. 5,249,581
[Patent Document 6] United State Patent No. 6,434,507
[Patent Document 7] United State Patent No. 7,166,114

The above mentioned navigation system is premised on the tool having been fixed relative to the actuator main body and, therefore, there has been found a problem that such navigation system is inapplicable to the remote controlled actuator of the type having the distal end member that can rotatably support the tool relative to the actuator main body in a fashion capable of altering in attitude thereof.

### SUMMARY OF THE INVENTION

The present invention is therefore intended to provide a navigation system applicable to a remote controlled actuator of a type, in which the attitude of a distal end member provided at a distal end portion of an elongated spindle guide section of a pipe-like configuration in appearance for supporting a tool can be altered by a remote control, which system is designed to enable the position of the tool to be estimated.

To describe a navigation system for a remote controlled actuator according to the present invention with the aid of reference numerals, used in the accompanying drawings, for facilitating a better understanding thereof, the remote controlled actuator includes an actuator main body 10, in which a base end of a spindle guide section 3 of an elongated configuration is connected with a drive unit housing 4a; a distal end member 2 fitted to a distal end portion of the spindle guide section 3 for pivotal movement about a pivot center O to enable it to be altered in attitude; a tool 1 rotatably supported by the distal end member 2; an attitude altering drive source 42 and a tool rotation drive source 41 both provided within the drive unit housing 4a for altering the attitude of the distal end member 2 and rotating the tool 1, respectively; and an operator unit 50 provided in the drive unit housing 4a for controlling respective operations of the drive sources 42 and 41 for maneuvering the attitude of the distal end member 2 and the rotation of the tool 1, to thereby estimate the position of a processing member 1a of the tool 1. The navigation system includes a marker detecting unit 8 for detecting respective positions and attitudes of a main body marker 7A, fitted to the drive unit housing 4a of the actuator main body 10, and a tool marker 7C provided at a predetermined relative position relative to the processing member 1a of the tool 1; a tool and tool marker relative position storage section 54 for storing a relative position of the processing member 1a of the tool relative to the tool marker 7C; a tool attitude detector 45 for detecting an angle of rotation of the distal end member about the pivot center O relative to the actuator main body 10; a tool marker relative position and attitude storage section 55; and a tool processing member position estimator 56.

For each angle of rotation of the distal end member 2 detected by the tool attitude detector 45, the tool marker relative position and attitude storage section 55 records the relative position and attitude of the tool marker 7C relative to the main body marker 7A, which have been calculated from the position and attitude of the main body marker 7A and the position and attitude of the tool marker 7C both detected by the marker detecting unit 8. The tool processing member position estimator 56 estimates the position of the processing member 1a of the tool 1 by estimating an absolute position and attitude of the tool marker 7C from the position and attitude of the main body marker 7A, detected by the marker detecting unit 8, and the relative position of the tool marker 7C relative to the main body marker 7A, which is obtained by checking the angle of rotation of the distal end member 2, detected by the tool attitude detector 45, with reference to the tool marker relative position and attitude storage section 55, and by checking a result of such estimation with stored information of the tool and tool marker relative position storage section 54 to thereby estimate the position of the processing member 1a of the tool 1.

The navigation system of the construction described above performs the following experiment prior to the procedure, using the remote controlled actuator 5 that is to be actually used for the procedure. That is to say, in a condition in which the tool marker 7C is provided at the predetermined relative position relative to the processing member 1a of the tool 1, the operator unit 50 is operated to alter the attitude of the distal end member 2 relative to the actuator main body 10 so that the angle of rotation of the distal end member 2 at that time can be detected by the tool attitude detector 45 and, at the same time, the respective positions and attitudes of the main body marker 7A and the tool marker 7C are detected by the marker detecting unit 8. Then, from the position and attitude of the main body marker 7A and the position and attitude of the tool marker 7C, the relative position and attitude of the tool marker 7C relative to the main body marker 7A are calculated. A series of those procedures are performed n times (n being an arbitrarily chosen integer) with the attitude of the distal member 2 changed and, then, for each angle of rotation of the distal end member 2, the relative position and attitude of the tool marker 7C relative to the main body marker 7A are recorded in the tool marker relative position and attitude storage section 55. Also, the relative position of the processing member 1a of the tool 1 relative to the tool marker 7C is stored by the tool and tool marker relative position storage section 54.

During the procedure, since the tool marker 7C is no longer necessary, it is removed so that the processing will not be disturbed. The angle of rotation of the distal end member 2, detected by the tool attitude detector 45, and the position and attitude of the main body marker 7A detected by the marker detecting unit 8 are inputted to the tool processing member position estimator 56. Those information changes from time to time when the remote controlled actuator 5 is manipulated. The tool processing member position estimator 56 estimates the absolute position and attitude of the tool marker 7C on each occasion from the position and attitude of the main body marker 7A and the relative position of the tool marker 7C relative to the main body marker 7A that is obtained by checking the rotational angle of the distal end member 2 with the tool marker relative position and attitude storage section 55. Yet, the position of the processing member 1a of the tool 1 is estimated by checking a result of such estimation with the stored information in the tool and tool marker relative position storage section 54. By so doing, the position of the processing member 1a of the tool 1 during the processing can be estimated relative to the remote controlled actuator 5 capable of altering the attitude of the distal end member 2 for the support of the tool 1 provided in the distal end portion of the spindle guide section 3.

In the present invention, it is recommended that the tool marker be fitted to a calibrating member removably mounted on the distal end member.

It is difficult to fit the tool marker 7C directly to the tool 1. Since the tool 1 and the distal end member 2 are held under a constant positional relationship, fitting of the tool marker 7C to the distal end member 2 through the calibrating member 58 makes it possible to provide the tool marker 7C at a predetermined relative position relative to the tool 1.

It is preferred that where the processing member 1a of the tool 1 is of a curved configuration, at least a portion of an outer surface thereof, which contains a tip end point Q1 located on a rotational center line CL of the tool 1, being convexed outwardly thereof, the calibrating member 58 has a calibrating point Q2 that is held in contact with the tip end point Q1 of the processing member 1a, a portion of the calibrating member 58 in proximate to the calibrating point Q2 having an outer surface 58a of a curved shape concaved along a curved outer surface of the processing member 1a.

At the time the tool marker 7C is to be fitted to the distal end member 2 through the calibrating member 58, by causing the tip end point Q1 of the tool 1 to contact the calibrating point Q2 of the calibrating member 58, the relative position of the tool marker 7C is determined relative to the tool 1. If the outer surface shape of that portion of the calibrating member 58 in proximate to the calibrating point Q2 is of the concave shape comprised of the curved surface 58a following the curved surface of the processing member 1a of the tool 1, the tip end point Q1 of the tool 1 can be accurately caused to contact the calibrating point Q2 of the calibrating member 58 and, therefore, the accuracy of the relative position of the tool marker 7C relative to the tool 1 can be increased.

In the present invention, each of the main body marker and the tool marker may be of a type capable of projecting or reflecting light and the marker detecting unit is of an optical type capable of receiving light from the main body marker and the tool marker. The optical marker detecting unit has a simplified structure.

In the present invention, the tool attitude detector 45 may be provided in the attitude altering drive source 42 or a drive system for transmitting an operation from the attitude altering drive source 42 to the distal end member 2 and is operable to output an electric signal corresponding to the attitude of the distal end member 2.

If the operation of the distal end member 2 is outputted in the form of the electrical signal, the transmission of information between the remote controlled actuator and a control system portion of the navigation system can be facilitated particularly where the remote controlled actuator and the control system portion are separated a distance from each other.

In the present invention, a display unit 52 may be provided for displaying images such that the tool processing member position estimator 56 is provided with an actuator display information generator 56a for calculating an actuator display information, which is information for displaying the position and attitude of the actuator main body 10 and the attitude of the distal end member 2, from various input information that is used in the estimation of the position of the tool 1, and then displaying such actuator display information on a screen of the display unit 52.

If the display unit 52 and the actuator display information generator 56a are employed, the position and attitude of the actuator main body 10 and the actuator display information, which is information for displaying the attitude of the distal end member 2, can be displayed on the screen of the display unit 52 and, therefore, the operator can readily recognize such information during the manipulation of the remote controlled actuator 5.

Where the display unit 52 and the actuator display information generator 56a are employed, the actuator display information generated by the actuator display information generator 56a may be information necessary to display the position and attitude of the actuator main body 10 and the attitude of the distal end member 2 on the screen of the display unit in the form of series of dots 60.

Display in the form of the dots 60 makes it possible for the operator to recognize visually and also facilitate the calculation taking place in the actuator display information generator 56a.

Also, the actuator display information generator 56a may be of a type generating, as the actuator display information, a graphic symbol 61 and then displaying the graphic symbol 61 on the screen of the display unit 52, which symbol 61 is representative of an external shape of the tool 1, the distal end member 2 and the actuator main body 10, which reflect the position and attitude of them, by means of a computer graphics.

If the graphic symbol 61 representative of the outer shape is displayed, the visual recognition can be further facilitated.

Furthermore, the actuator display information generated by the actuator display information generator 56a may be information in the form of a numeral on the screen of the display unit 52.

In any case, by displaying on the screen of the display unit 52, the position and attitude of the actuator main body 10 and the actuator display information which is the information for displaying the position of the distal end member 2, the operator can be informed of such information.

The remote controlled actuator is preferably constructed as follows. That is to say, the distal end member rotatably supports the spindle for holding the tool; the spindle guide section includes a rotatable shaft for transmitting rotation of the tool rotation drive source within the drive unit housing to the spindle and an attitude altering member for altering the attitude of the distal end member by selectively advancing or retracting by means of the attitude altering drive source within the drive unit housing in a condition with the tip end held in contact with the distal end member.

If the remote controlled actuator is of the structure described above, the rotation of the tool rotation drive source within the drive unit housing is transmitted to the spindle through the rotary shaft accommodated within the spindle guide section and the attitude altering member arranged within the spindle guide section is selectively advanced or retracted by the attitude altering drive source within the drive unit housing, to thereby alter the attitude of the distal end member. Accordingly, the rotation of the tool and the alteration of the attitude of the distal end member can be accomplished by remote control.

### BRIEF DESCRIPTION OF THE DRAWINGS

In any event, the present invention will become more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims. In the accompanying drawings, like reference numerals are used to denote like parts throughout the several views, and:
Fig. 1 is a schematic structural diagram showing a condition in which a procedure takes place with a navigation system for a remote controlled actuator according to a first preferred embodiment of the present invention;
Fig. 2A is a schematic structural diagram showing a condition during the testing of the navigation system;
Fig. 2B is a fragmentary enlarged view showing a portion of the navigation system shown in Fig. 2A;
Fig. 3A is a sectional view showing a distal end member and a spindle guide section both employed in the remote controlled actuator according to the first preferred embodiment of the present invention;
Fig. 3B is a cross sectional view taken along the line IIIB-IIIB in Fig. 3A;
Fig. 3C is a diagram showing a coupling structure between the distal end member and a rotary shaft;
Fig. 4A is a side view showing a tool rotation drive mechanism and an attitude altering drive mechanism both employed in the remote controlled actuator;
Fig. 4B is a cross sectional view taken along the line IVB-IVB in Fig. 4A;
Fig. 5 is a block diagram showing a control system of the navigation system;
Fig. 6 is a diagram showing a structure of a tool marker relative position and attitude storage section employed in the navigation system;
Fig. 7A is a diagram showing a tool, the distal end member and the spindle guide section, all employed in the remote controlled actuator;
Fig. 7B is a diagram showing the spindle guide section of a different shape;
Fig. 8A is a diagram showing the tool, the distal end member and the spindle guide section, all employed in the remote controlled actuator;
Fig. 8B is a diagram showing the tool of a different type;
Fig. 9 is a diagram showing an example of an image displayed on a display unit of the navigation system;
Fig. 10 is a diagram showing a different example of the image displayed on the display unit of the navigation system;
Fig. 11A is a sectional view showing the distal end member and the spindle guide section, all employed in the remote controlled actuator designed in accordance with a second preferred embodiment of the present invention;
Fig. 11B is a cross sectional view taken along the XIB-XIB in Fig. 11A;
Fig. 12A is a sectional view showing the distal end member and the spindle guide section, all employed in the remote controlled actuator designed in accordance with a third preferred embodiment of the present invention;
Fig. 12B is a cross sectional view taken along the XIIB-XIIB in Fig. 12A;
Fig. 13 is a sectional view similar to Fig. 4B, which shows the tool rotation drive mechanism and the attitude altering drive mechanism both employed in the remote controlled actuator;
Fig. 14 is a diagram showing a schematic structure of the navigation system for the remote controlled actuator according to a mode of application of the present invention;
Fig. 15A is a sectional view showing the distal end member and the spindle guide section both employed in the remote controlled actuator;
Fig. 15B is a cross sectional view taken along the line XVB-XVB in Fig. 15A;
Fig. 15C is a diagram showing the coupling structure between the distal end member and the rotary shaft;
Fig. 15D is a view showing a housing for the distal end member as viewed from the side of a base or proximal end;
Fig. 16 is a sectional view showing the tool rotation drive mechanism and the attitude altering drive mechanism both employed in the remote controlled actuator;
Fig. 17 is a diagram showing a first structural example of a marker support mechanism for a rotational angle detection marker;
Fig. 18A is a sectional view showing a second structural example of the marker support mechanism for the rotational angle detection marker;
Fig. 18B is a diagram as viewed in a direction along the arrow XVIIIB in Fig. 18A;
Fig. 18C is a cross sectional view taken along the line XVIIIC-XVIIIC in Fig. 18A;
Fig. 19 is a block diagram showing the control system of the navigation system;
Fig. 20A is a diagram showing the tool, the distal end member and the spindle guide section all employed in the remote controlled actuator;
Fig. 20B is a diagram showing the spindle guide section of a further different shape;
Fig. 21 is a diagram showing a structure of an actuator shape storage section employed in the navigation system;
Fig. 22A is a diagram showing the tool, the distal end member and the spindle guide section all employed in the remote controlled actuator;
Fig. 22B is a diagram showing the tool of a further different type;
Fig. 22C is a diagram showing the tool of a still further different type;
Fig. 23 is a diagram showing a structure of a portion of the actuator shape storage section in the navigation system;
Fig. 24 is a perspective view showing a drive unit housing in a different navigation system; and
Fig. 25 is a sectional view showing the tool rotation drive mechanism and the attitude altering drive mechanism both employed in the remote controlled actuator in the navigation system.

### DESCRIPTION OF PREFERRED EMBODIMENTS

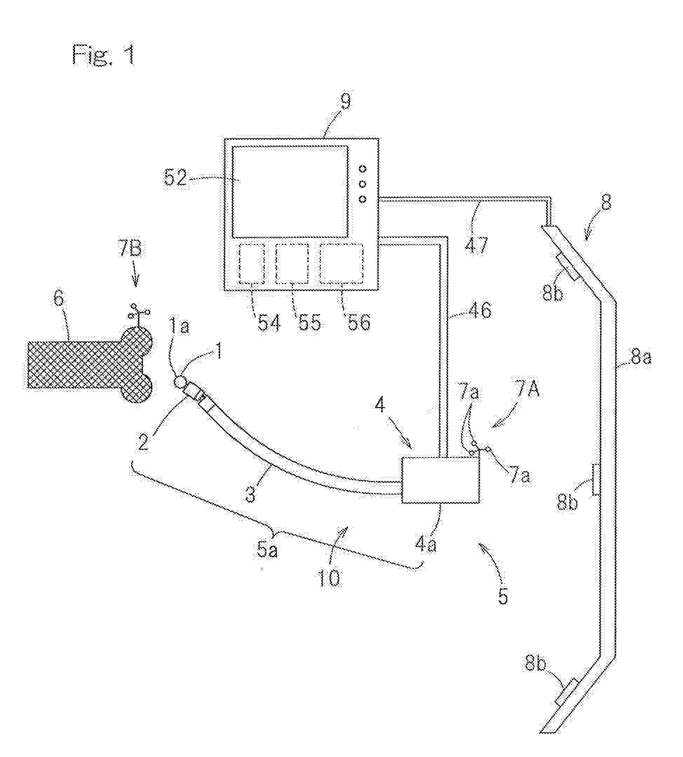

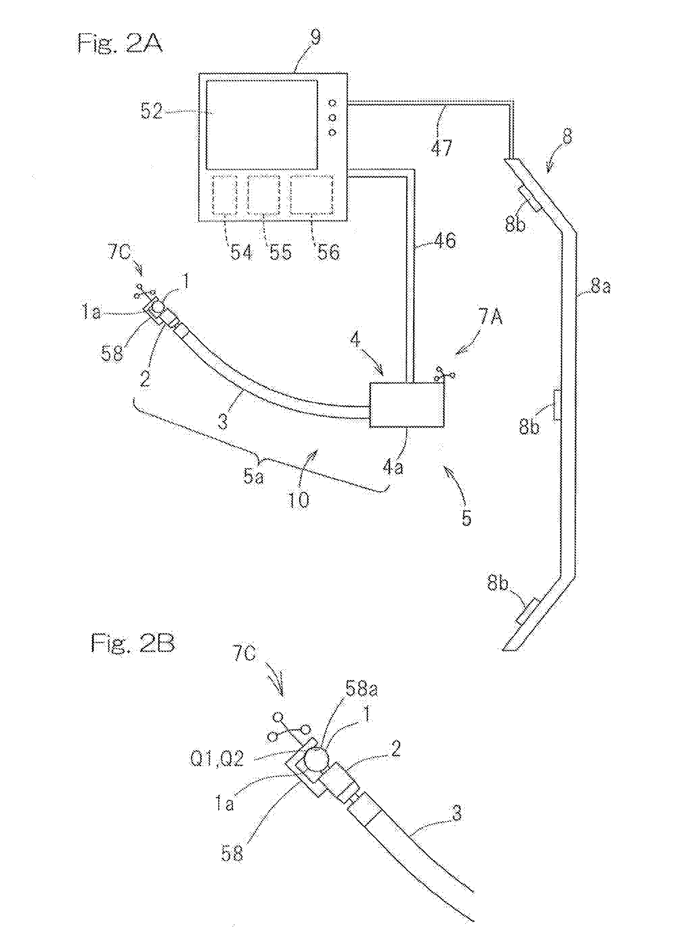

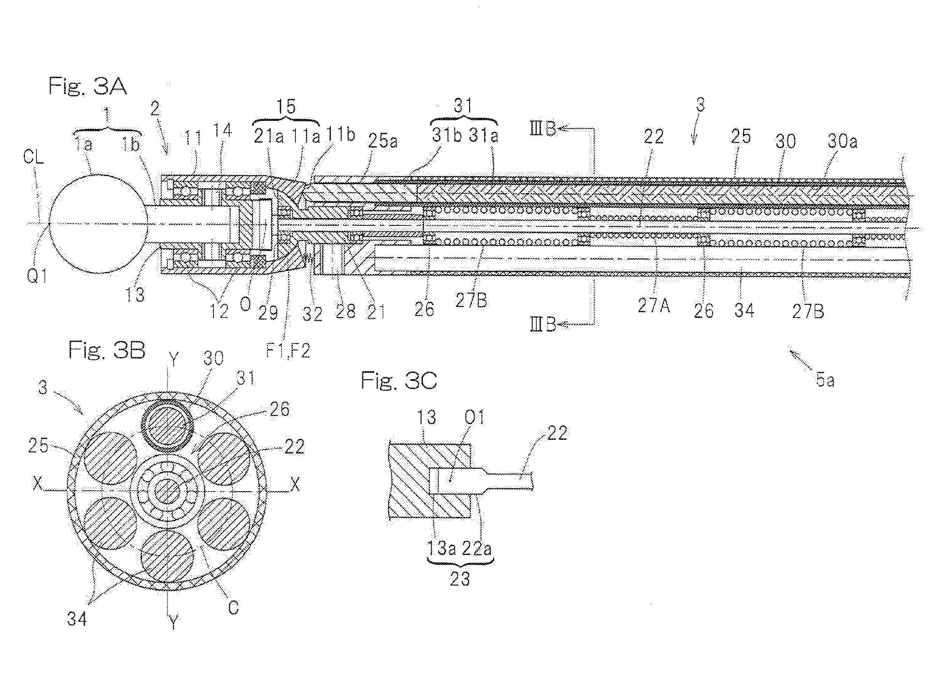

Fig. 1 and Figs. 2A and 2B illustrate a schematic structure of a navigation system for a remote controlled actuator designed in accordance with a first preferred embodiment of the present invention. In particular, Fig. 1 illustrates the condition during a procedure taking place and Figs. 2A and 2B illustrates the condition during the test prior to the procedure taking place. The illustrated navigation system is utilized in association with the remote controlled actuator 5 of a type capable of navigating the rotation and the attitude of a tool 1 by remote control and includes a marker detecting unit 8 for detecting the positions and the attitudes of a marker 7A fitted to the remote controlled actuator 5, a marker 7B fitted to a to-be-processed object 6 and a marker 7C (best shown in Fig. 2A) that is held at a predetermined relative position relative to a processing member 1a (best shown in Fig. 3A) of the tool 1, and a navigation and maneuvering computer 9 for concurrently controlling the navigation system and the operation of the remote controlled actuator 5.

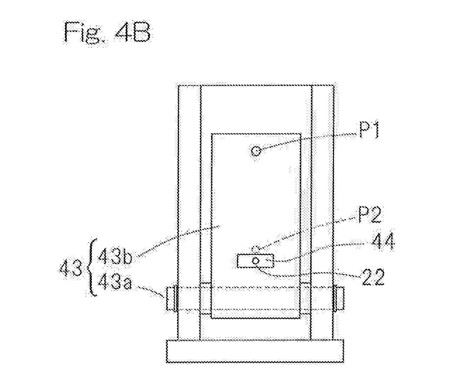

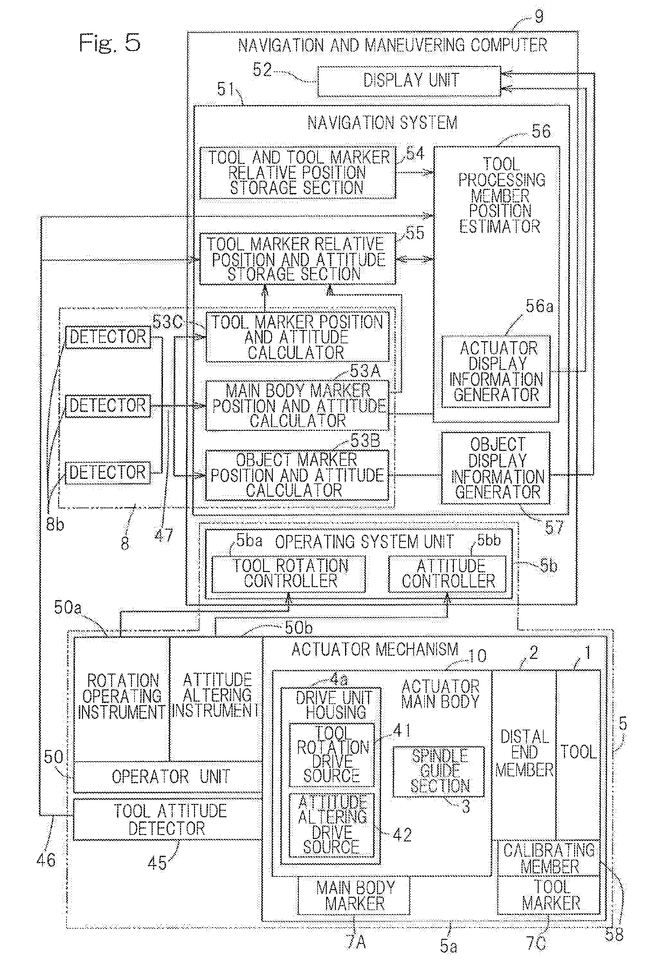

The remote controlled actuator 5 includes an actuator mechanism 5a, best shown in Fig. 1 and Fig. 2A, and an operating system unit 5b, best shown in Fig. 5, within the navigation and maneuvering computer 9. Hereinafter, the details of the actuator mechanism 5a will be described with particular reference to Fig. 1 to Figs. 4A and 4B. It is, however, to be noted that while Fig. 3A illustrates a spindle guide section 3 that extends linearly, the spindle guide section 3 can have a basically identical structure regardless of whether it has a linear shape as shown in Fig. 3A, it has a curved shape as shown in Fig. 2A or whether it has any other shape.

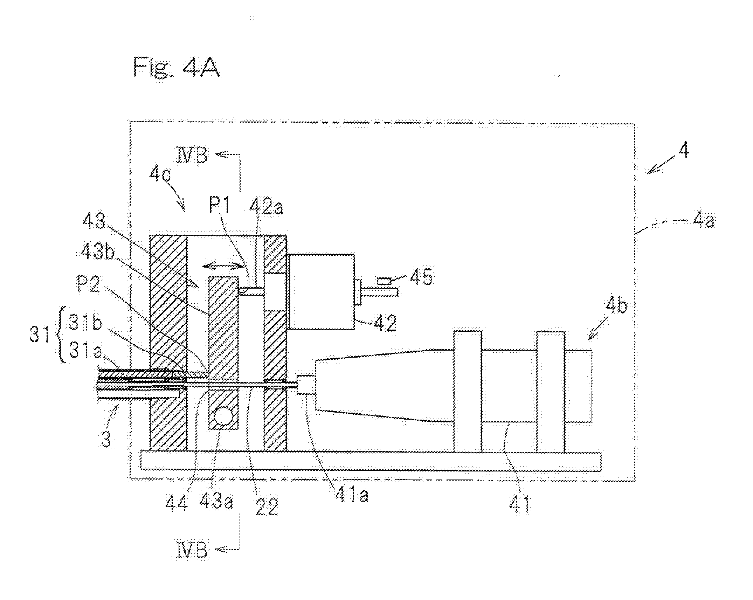

Referring now to Fig. 1 and Figs. 2A and 2B, the actuator mechanism 5a includes a distal end member 2 for holding a rotary tool 1, the elongated spindle guide section 3 of a pipe-like appearance having its distal end to which the distal end member 2 is fitted for alteration in attitude, and a drive unit housing 4a to which a proximal end of the spindle guide section 3, opposite to the above mentioned distal end, is connected. The drive unit housing 4a cooperates with a built-in tool rotation drive mechanism 4a, best shown in Fig. 4A, and a similarly built-in attitude altering drive mechanism 4c to form a drive unit 4. Also, the spindle guide section 3 and the drive unit 4 altogether constitute an actuator main body 10. The drive unit housing 4a is provided with an operator unit 50 that is made up of a rotation operating instrument 50a, best shown in Fig. 5, for rotating the tool 1 by controlling the operation of the tool rotation drive mechanism 4b and an attitude altering instrument 50b, also best shown in Fig. 5, for effecting alteration of the attitude of the distal end member 2 by controlling the operation of the attitude altering drive mechanism 4c.

As shown in Fig. 3A, the tool 1 is made up of the processing member 1a and a shank 1b. In the embodiment now under discussion, the processing member 1a is of a spherical shape. The distal end member 2 includes a generally or substantially cylindrical housing 11 and a spindle 13 rotatably accommodated within such cylindrical housing 11 through a pair of bearings 12. The spindle 13 is of a tubular shape having a distal side opening and having a hollow defined therein, and a tool 1 is drivingly coupled with the spindle 13. Specifically, a shank 1b of the tool 1 is inserted into the hollow of the spindle 13 in a removable fashion and is then coupled with such spindle 13 by means of a stop pin 14 for rotation together with the spindle 13. The distal end member 2 of the structure described above is coupled with a distal end of the spindle guide section 3 through a distal end member connecting unit 15. The distal end member connecting unit 15 is means for supporting the distal end member 2 for displacement in attitude and is comprised of a spherical bearing. More specifically, the distal end member connecting unit 15 includes a guided member 11a in the form of an inner diameter reduced portion at a base end of the housing 11, and a guide member 21a in the form of a collar integral with a constraint member 21 fixed to the tip of the spindle guide section 3. The guided member 11a and the guide member 21a have respective guide faces F1 and F2 that are held in sliding contact with each other, and those guide faces F1 and F2 have respective centers of curvature lying at a point O on the center line or longitudinal axis CL of the tool 1 and the spindle 13, having their diameters being reduced towards the base end of the spindle 13. Accordingly, not only can the distal end member 2 be immovably constrained relative to the spindle guide section 3, but it can also be supported for displacement in attitude so that the attitude of the distal end member 2 can be altered. It is to be noted that since in this example, the distal end member 2 can have its attitude altered about a lateral X-axis passing through the center of curvature O, the guide faces F1 and F2 may be a cylindrical surface having a longitudinal axis represented by the X-axis passing through the center of curvature O.

The spindle guide section 3 includes a rotary shaft 22 for transmitting a rotational force exerted by a tool rotation drive source 41 accommodated within the drive unit housing 4a (Fig. 4A). In the illustrated example, the rotary shaft 22 is employed in the form of a wire capable of undergoing deformation to a certain extent. Material for the wire includes, for example, metal, resin or glass fiber. The wire may be either a single wire or a stranded wire. As best shown in Fig. 3C, the spindle 13 and the rotary shaft 22 are connected together by means of a universal joint 23 for transmitting rotation from the rotary shaft 22 to the spindle 13. The universal joint 23 is made up of a groove 13a, defined in a closed base end of the spindle 13, a projection 22a defined in a distal end of the rotary shaft 22 and engageable in the groove 13a. The center O1 of joint between the groove 13a and the projection 22a is located at the same position as the centers of curvature O of the guide faces F1 and F2. It is, however, to be noted that the rotary shaft 22 and the projection 22a may be respective members separate from each other.

The spindle guide section 3 includes an outer shell pipe 25 forming an outer shell of the spindle guide section 3 and the rotary shaft 22 referred to above is positioned at the center of this outer shell pipe 25. The rotary shaft 22 so positioned is rotatably supported by a plurality of rolling bearings 26 positioned spaced a distant apart from each other in a direction axially of the spindle guide section 3. Spring elements 27A and 27B for generating a preload on the corresponding rolling bearing 26 are disposed between the neighboring rolling bearings 26. Each of those spring elements 27A and 27B is employed in the form of, for example, a compression spring. There are the spring element 27A for inner ring for generating the preload on the inner ring of the rolling bearing 26 and the spring element 27B for outer ring for generating the preload on the outer ring of the rolling bearing 26, and the both are arranged alternately relative to each other. The constraint member 21 referred to previously is fixed to a pipe end portion 25a of the outer shell pipe 25 by means of a fixing pin 28 and has its distal end inner peripheral portion supporting the distal end of the rotary shaft 22 through a rolling bearing 29. It is, however, to be noted that the pipe end portion 25a may be a member separate from the outer shell pipe 25 and may then be connected with the outer shell pipe 25 by means of, for example, welding.

A single guide pipe 30 open at opposite ends thereof is provided between an inner diametric surface of the outer shell pipe 25 and the rotary shaft 22, and an attitude altering member 31, made up of a wire 31a and pillar shaped pins 31b at opposite ends, is axially movably inserted within a guide hole 30a, which is an inner diametric hole of the guide pipe 30. One of the pillar shaped pins 31b, which is on the side of the distal end member 2, has its tip representing a spherical shape and is held in contact with a base end face of the distal end member housing 11. The base end face 11b of the housing 11, which defines a surface of contact between the distal end member 2 and the attitude altering member 31, for the distal end member 2 is so shaped as to represent an inclined face such that an outer peripheral edge thereof is closer to the spindle guide section 3 than a center portion thereof. Similarly, as best shown in Fig. 4A, the other of the pillar shaped pins 3 1b, that is, the pillar shaped pin 3 1b on the side of the drive unit housing 4a has its tip representing a spherical shape and held in contact with a side face of a lever 43 as will be described in detail later.

It is to be noted that the use of the pillar shaped pins 31b may be dispensed with, leaving only the signal wire 31a to constitute the attitude altering member 31.

At a position spaced 180° in phase from a peripheral position where the attitude altering member 31 referred to above is positioned, a restoring elastic member 32, which is in the form of, for example, a compression spring, is provided between the base end face of the housing 11 for the distal end member 2 and a tip end face of the outer shell pipe 25 of the spindle guide section 3. This restoring elastic member 32 has a function of biasing the distal end member 2 towards the side of a predetermined attitude.

Also, as best shown in Fig. 3B, between the inner diametric surface of the outer shell pipe 25 and the rotary shaft 2, a plurality of reinforcement shafts 34 are arranged separate from the guide pipe 30 and on the pitch circle C of the same diameter as the guide pipe 30. Those reinforcement shafts 34 are used to secure the rigidity of the spindle guide section 3. The guide pipe 30 and the reinforcement shafts 34 are arranged equidistantly relative to each other around the rotary shaft 22. The guide pipe 30 and the reinforcement shafts 34 are held in contact with the inner diametric surface of the outer shell pipe 25 and respective outer peripheral surfaces of the rolling bearings 26. In this manner, the outer diametric surfaces of those rolling bearings 26 are supported.

The tool rotation drive mechanism 4b includes the tool rotation drive source 41 referred to previously. The tool rotation drive source is in the form of, for example, an electrically driven motor having its output shaft 41a coupled with a base or proximal end of the rotary shaft 22.

The attitude altering drive mechanism 41 includes an attitude altering drive source 42. This attitude altering drive source 42 is in the form of, for example, an electrically operated linear actuator and had an output rod 42a capable of moving leftwards or rightwards, as viewed in Fig. 4A, the movement of such output rod 42a being transmitted to the attitude altering member 31 through a lever mechanism 43, which is a force transmitting mechanism. It is to be noted the attitude altering drive source 42 may be a rotary motor. The amount of actuation of the attitude altering drive source 42 is detected by a tool attitude detector 45. A detection signal outputted from this tool attitude detector 45 is supplied to a tool processing member position estimator (best shown in Fig. 5) of the navigation and maneuvering computer 9 through an actuator electric cable 46 (best shown in Fig. 1 and Fig. 2A).

The lever mechanism 43 includes a pivot lever 43b pivotable about a support pin 43a and is so designed and so configured as to allow a force of the output rod 42a to work on a working point P1 of the lever 43b, which is spaced a long distance from the support pin 43a, and as to apply a force to the attitude altering member 31 at a force point P2, which is spaced a short distance from the support axis 43a, wherefore an output of the attitude altering drive source 42 can be increased and then transmitted to the attitude altering member 31. Since the use of the lever mechanism 43 is effective to enable a large force to be applied to the attitude altering member 31 even in the linear actuator of a low output capability, the linear actuator can be downsized. The rotary shaft 22 extends through an opening 44 defined in the pivot lever 43b. It is to be noted that instead of the use of the attitude altering drive source 42 or the like, the attitude of the distal end member 2 may be manually operated from a remote site (by remote control).

The marker detecting unit 8 referred to previously and best shown in Figs. 1 and 2A makes use of markers 7A and 7B (both best shown in Fig. 1) and 7C (best shown in Fig. 2A) as respective objects to be detected and includes three marker detectors 8b, which are supported by a detector support body 8a, and marker position and attitude calculators 53A, 53B and 53C (best shown in Fig. 5) built in the navigation and maneuvering computer 9. The main body marker 7A is fitted to the drive unit housing 4a forming a part of the actuator main body 10 and the object marker 7B is fitted to the object to be processed 6 such as, for example, a patient's bone or the like. On the other hand, the manner of fitting the tool marker 7C will be described later. In association with the respective marker detectors 8b in the marker detecting unit 8, the actuator, object and tool markers 7A, 7B and 7C are provided with respective sets of three light reflectors 7a. Those three light reflectors 7a are differentiated in position.

Each of the marker detectors 8b is of an optical type and is so designed and so configured as to project a detection beams towards the light reflectors 7a of each of the markers 7A, 7B and 7C and then receive rays of light reflected from those light reflectors 7a. Respective detection signals of those marker detectors 8b are supplied to respective marker position and attitude calculators 53A, 53B and 53C (best shown in Fig. 5) in the navigation and maneuvering computer 9 through a wiring system (not shown), built in the detector support body 8a, and a marker detector electric cables 47. It is, however, to be noted that the use of three light projectors (not shown) may be provided respectively in the markers 7A, 7B and 7C so that detection beams projected from those light projectors can be received by the individual detectors 8b. The use of the marker detectors 8b of an optical type as discussed above is effective to allow the marker detecting unit 8 to be assembled portable. It is, however, also to be noted that each of the marker detectors 8b may not necessarily be of an optical type and may be of, for example, a magnetic type.

As shown in Fig. 5, the navigation and maneuvering computer 9 includes the operating system unit 5b referred to previously, a navigation system unit 51 and a display unit 52. The operating system unit 5b and the navigation system unit 51 are comprised of hardware of the navigation and maneuvering computer 9 and a software program executed thereby, or comprised of a further addition of an electronic circuit.

The operating system unit 5b is made up of a tool rotation controller 5ba and an attitude controller 5bb. The tool rotation controller 5ba provides an output to a motor driver (not shown) in response to an input from a rotation operating instrument 50a so as to drive the tool rotation drive source 41. The attitude controller 52b provides an output to the motor driver (not shown) in response to an input from an attitude altering instrument 50b to thereby drive the attitude altering drive source 42.

The navigation system unit 51 includes marker position and attitude calculators 53A, 53B and 53C for the main body, the object to be processed and the tool, respectively, a tool and tool marker relative position storage section 54, a tool marker relative position and attitude storage section 55, a tool processing member position estimator 56. The tool processing member position estimator 56 includes an actuator display information generator 56a. Also, separate from those, the navigation system unit 51 includes an object display information generator 57.

The marker position and attitude calculators 53A, 53B and 53C calculate respective positions and attitudes of the actuator, object and tool markers 7A, 7B and 7C in reference to associated detection signals from the three individual detectors 8b of the marker detecting unit 8. When each of the light reflector 7a of the main body, object and tool markers 7A, 7B and 7C and the individual detectors 8b is employed in three or more in number, the three dimensional position and attitude of each of the markers 7A, 7B and 7C can be determined. The position and attitude of the main body marker 7A are synonymous to the position and attitude of the actuator main body 10. The position and attitude of the object marker 7B are synonymous to the position and attitude of the to-be-processed object 6.

The tool and tool marker relative position storage section 54 is operable to store the relative position of the processing member 1a of the tool 1 relative to the tool marker 7C and, in the case of the embodiment now under discussion, the relative position of a tip point Q1, as will be described later, of the processing member 1a is stored.

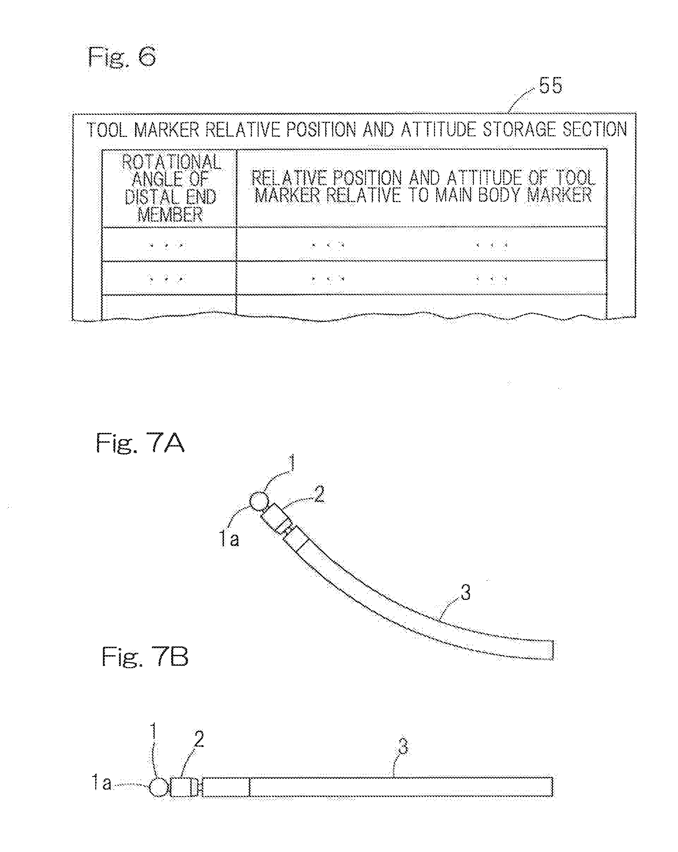

Referring to Fig. 6, the tool marker relative position and attitude storage section 55 is of a kind, in which the relative position and attitude of the tool marker 7C relative to the main body marker 7A are recorded for each angle of rotation of the distal end member 2 detected by the tool attitude detector 45 shown in Fig. 5. The relative position and attitude of the tool marker 7C relative to the main body marker 7A are calculated from the position and attitude of the tool marker 7C and the position and attitude of the main body marker 7A, which are detected by the marker detectors 8.

The angle of rotation of the distal end member 2 and the relative position and attitude of the tool marker 7C relative to the main body marker 7A, both of which are recorded in the tool marker relative position and attitude storage section 55, are determined by means of a series of experiments conducted with the use of the actuator mechanism 5a which is actually used in processing. More specifically, those series of experiments are conducted as shown in Fig. 2A. In other words, while the tool marker 7C is provided at a predetermined relative position relative to the processing member 1a of the tool 1, the attitude altering instrument 50b best shown in Fig. 5 is manipulated to cause the distal end member 2 to alter the attitude of the distal end member 2 relative to the actuator main body 10 and the angle of rotation of the distal end member 2 at that time is detected by the tool attitude detector 45 (best shown in Fig. 5) and, at the same time, the positions and the attitudes of the main body marker 7A and the tool marker 7C at that time, respectively are detected by the marker detecting unit 8. Then, from the position and attitude of the main body marker 7A and the position and attitude of the tool marker 7C, the relative position and attitude of the tool marker 7C relative to the main body marker 7A are calculated. This work is repeated an arbitrarily chosen number of times with the attitude of the distal end member 2 changed and, for each of the angle of rotation of the distal end member 2, the relative position and attitude of the tool marker 7C relative to the main body marker 7A are recorded in the tool marker relative position and attitude storage section 55.

As shown in a fragmentary enlarged view in Fig. 2B, the tool marker 7C is fitted to a removable calibrating member 58 at the distal end member 2 and, during the experiments, the calibrating member 58 equipped with the tool marker 7C is fixedly mounted on the distal end member 2. At this time, a point of calibration Q2 of the calibrating member 58 is held in contact with the tip point Q1 of the processing member 1a. As shown in Fig. 3A, the tip point Q1 is represented by a point on the rotational center line CL on an outer peripheral surface of the processing member 1a. As best shown in Fig. 2B, a portion of the calibrating member 58 in proximate to the point of calibration Q2 has an outer surface represented by a curved surface 58a that is so concaved as to follow an outer surface of the processing member 1a of the tool 1. In such case, the curved surface 58a is a spherical surface.

It is difficult to fit the tool marker 7C directly to the tool 1, but the use of the calibrating member 58 of the type referred to above makes it easy to fit the tool marker 7C to the tool 1. Since the tool 1 and the distal end member 2 are held in a predetermined positional relationship at all times, the contact of the tip point Q1 of the tool 1 with the calibration point Q2 of the calibrating member 58 is effective to position the tool marker 7C at the predetermined relative position relative to the processing member 1a of the tool 1. Also, when the shape of the outer surface of that portion of the calibrating member 58 in the vicinity of the calibration point Q2 is rendered to be the curved surface 58a of the concave shape as hereinabove described, the tip point Q1 of the tool 1 can be accurately held in contact with the calibration point Q2 of the calibrating member 58, thus resulting in an increased accuracy of the relative position of the processing member 1a of the tool 1 relative to the tool marker 7C.

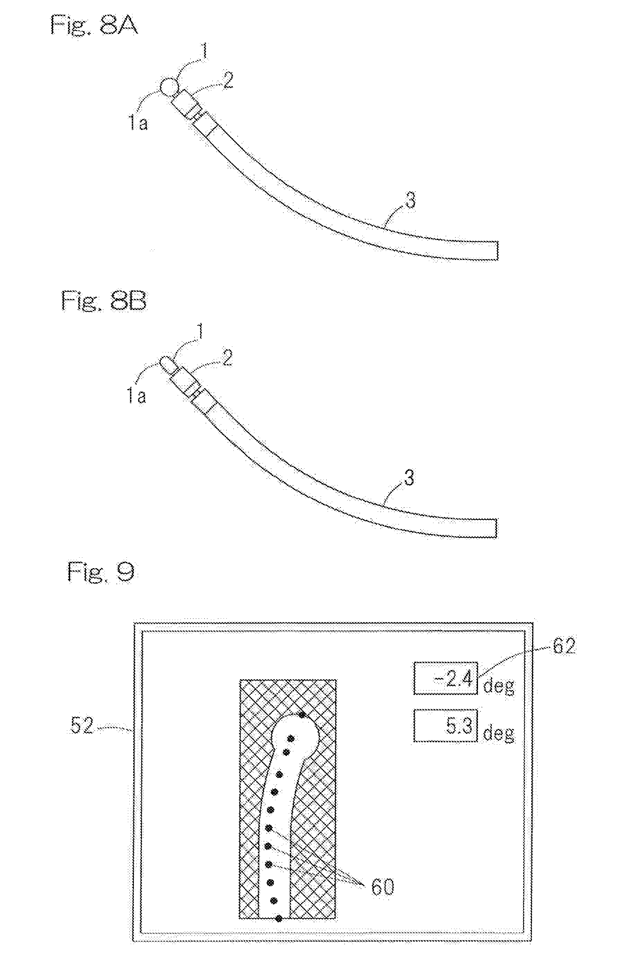

The relationship between the position and attitude of the main body marker 7A and the position and attitude of the tool marker 7C depends on the shape of the spindle guide section 3. For example, it differs depending on whether the spindle guide section 3 employed has a curved shape as shown in Fig. 7A or whether it has a straight shape as shown in Fig. 7B. Even where the spindle guide section 3 is, for example, artificially deformed, that before the deformation and that after the deformation differs from each other. Similarly, the above described relation differs depending on the kind of the tool 1 used. For example, it differs depending on whether the processing member 1a of the tool 1 has a spherical shape as shown in Fig. 8A or whether it has a pillar shaped shape as shown in Fig. 8B. For these reasons, when one or both of the spindle guide section 3 and the tool 1 is/are replaced, or when the spindle guide section 3 is deformed, the series of experiments referred to have to be executed in a manner similar to that described hereinabove so as to determine the relative position and attitude of the tool marker 7C relative to the main body marker 7A shown in Fig. 2A.

The tool processing member position estimator 56 referred to above and shown in Fig. 5 estimates the absolute position and attitude of the tool marker 7C in reference to the position and attitude of the main body marker 7A, detected by the marker detecting unit 8, and the relative position of the tool marker 7C relative to the main body marker 7A, which are obtained as a result that the angle of rotation of the distal end member 2, detected by the tool attitude detector 45, are checked by the tool marker relative position and attitude storage section 55 and, at the same time, estimates the position of the processing member 1a of the tool 1, shown in Fig. 2A, by checking a result of such estimation with stored information of the tool and tool marker relative position storage section 54.

In other words, by detecting the angle of rotation of the distal end member 2 with the tool attitude detector 45, shown in Fig. 5, and then by checking the detected angle of rotation with the tool marker relative position and attitude storage section 55, the relative position of the tool marker 7C relative to the main body marker 7A is determined. At this time, the actual measurement of the angle of rotation of the distal end member 2 detected by the tool attitude detector 45 does not match with the angle of rotation of the distal end member 2 recorded in the tool marker relative position and attitude storage section 55, but the relative position of the tool marker 7C relative to the main body marker 7A may be determined using the most approximate value or by calculation based on the relative position of the tool marker 7C relative to the main body marker 7A that is delivered from a plurality of values approximate to the actual measurement. Then, when the above described relative position so determined as above is checked with the position and attitude of the main body marker 7A detected by the marker detecting unit 8, the absolute position and attitude of the tool marker 7C can be estimated.

Since the calibration point Q2 of the calibrating member 58 relative to the tool marker 7C as shown in Fig. 2B is held at the predetermined relative position, the position of the calibration point Q2 can be ascertained if the absolute position and attitude of the tool marker 7C is made available. Since the calibration point Q2 is at the same position as the tip point Q1, the position of the processing member 1a of the tool 1 can be ascertained. Accordingly, the position of the processing member 1a of the tool 1 during the procedure can be estimated relative to the remote controlled actuator 5 of the type in which the attitude of the distal end member 2 for the support of the tool, which is provided in the distal end portion of the spindle guide section 3, can be altered by remote control.

The actuator display information generator 56a referred to above and shown in Fig. 5 is operable to calculate an actuator display information, which is information for displaying the position and attitude of the actuator main body 10 and the attitude of the distal end member 2 from various pieces of information used to estimate the position of the processing member 1a of the tool 1 shown in Fig. 1 and then to display a result of such calculation on a screen of the display unit 52. Also, the object display information generator 57 shown in Fig. 5 is operable to calculate an object display information, which is information on the position and attitude of the object marker 7B determined by the marker position and attitude calculator 54B and then to display a result of such calculation on the screen of the display unit 52.

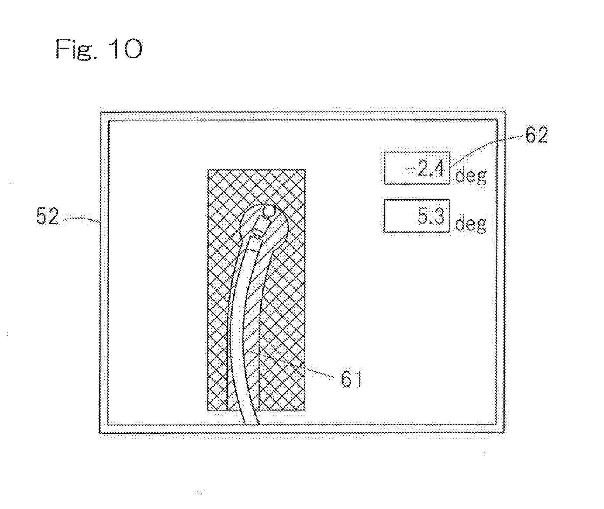

More specifically, as best shown in Fig. 9, the actuator display information and the object display information, both referred to above, that is, the position and attitude of the actuator main body 10, the attitude of the distal end member 2 and the position and attitude of the to-be-processed object 6 are displayed in the form of a plurality of dots 60. Fig. 9 illustrates respective positions of the spindle guide section 3 and the distal end member 2 being displayed in the form of the dots 60 spaced a predetermined distance from each other. Alternatively, as best shown in Fig. 10, using a computer graphics or the like, based on shape information of various portion of the actuator main body 10 already stored in the navigation and maneuvering computer 9, a representation 61 is displayed, which represents respective contours of the position and attitude of the spindle guide section 3, the distal end member 2, the tool 1 and the to-be-processed object 6. Yet, the actuator display information and the object display information may be displayed on display windows 62 in terms of numerical representations together with the dots 60 and the graphic symbol 61 as shown in Figs. 9 and 10. In the illustrated example, there is illustrated a condition in which the attitude of the distal end member 2 is displayed on the display windows 62. It is preferred that information other than the attitude of the distal end member 2 can also be selectively displayed.

Hereinafter, the operation of the remote controlled actuator 5 of the structure shown in Fig. 1 will be described.

During the procedure, the calibrating member 58 provided with the tool marker 7C best shown in Fig. 2A is removed from the distal end member 2. When starting from this condition, the tool rotation drive source 41 best shown in Fig. 4A is driven, the rotational force is transmitted to the spindle 13 through the rotary shaft 22 best shown in Fig. 3A so as to rotate the tool 1 together with the spindle 13. By the tool 1 then rotating, the bone or the like is cut. The rotational speed of the tool 1 can be arbitrarily set by means of the rotation operating instrument 50a (shown in Fig. 5).

The attitude altering drive source 42 (shown in Fig. 4A) is driven to alter the attitude of the distal end member 2 by remote control. By way of example, if the attitude altering member 31 is advanced by the attitude altering drive source 42 in a direction towards the tip or distal side, the housing 11 for the distal end member 2 is pressed by the attitude altering member 31 with the distal end member 2 consequently altered in attitude along the guide faces F1 and F2 so that the tip or distal side can be oriented downwardly as viewed in Fig. 3A. If the attitude altering member 31 is conversely retracted by the attitude altering drive source 42, the housing 11 for the distal end member 2 is pressed backwardly by the effect of the elastic repulsive force exerted by the restoring elastic member 32 and, consequently, the distal end member 2 is altered in attitude along the guide faces F1 and F2 so that the tip or distal side can be oriented upwardly as viewed in Fig. 3A. At this time, a pressure from the attitude altering member 31, the elastic repulsive force from the restoring elastic member 32 and a reactive force from the constraint member 21 are applied to the distal end member connecting unit 15 and, depending on the balance of those applied forces, the attitude of the distal end member 2 is determined. For this reason, the attitude of the distal end member 2 can be properly controlled by remote control.

Since the attitude altering member 31 is inserted through the guide hole 30a, the attitude altering member 31 can properly act on the distal end member 2 at all times without being accompanied by displacement in position in a direction perpendicular to the lengthwise direction thereof and the attitude altering operation of the distal end member 2 can therefore be performed accurately. Also, since the attitude altering member 31 is comprised of mainly the wire 31a and has a flexible property, the attitude altering operation of the distal end member 2 is carried out accurately even though the spindle guide section 3 is curved. In addition, since the center of the junction between the spindle 13 and the rotary shaft 22 lies at the same position as the respective centers of curvature O of the guide faces F1 and F2, no force tending to press and pull will not act on the rotary shaft 22 as a result of the alteration of the attitude of the distal end member 2 and the distal end member 2 can be smoothly altered in attitude.

The remote controlled actuator 5 of the foregoing construction is utilized in grinding the femoral marrow cavity during, for example, the artificial joint replacement surgery and during the surgery, it is used with the distal end member 2 in its entirety or a part thereof inserted into the body of a patient. Because of this, if the distal end member 2 can be altered in attitude by remote control, the bone can be processed in a condition with the tool 1 maintained in a proper attitude at all times and the opening for insertion of the artificial joint can be finished accurately and precisely.

There is the necessity that the rotary shaft 22 and the attitude altering member 31 are provided in a protected fashion. In this respect, the spindle guide section 3, which is elongated in shape, is provided with the rotary shaft 22 at the center of the outer shell pipe 25 and the guide pipe 30, accommodating therein the attitude altering member 31, and the reinforcement shafts 34, all of these are arranged in the circumferential direction and between the outer shell pipe 25 and the rotary shaft 22. Accordingly, the rotary shaft 22 and the attitude altering member 31 can be protected and the interior can be made hollow to thereby reduce the weight without sacrificing the rigidity. Also, the arrangement balance as a whole is rendered good.

Since, as shown in Fig. 3B, the outer diametric surfaces of the rolling bearings 26 supporting the rotary shaft 22 are supported by the guide pipe 30 and the reinforcement shafts 34, the outer diametric surfaces of the rolling bearings 26 can be supported with no need to use any extra member. Also, since the preload is applied to the rolling bearings 26 by means of the spring elements 27A and 27B as shown in Fig. 3A, the rotary shaft 22 comprised of the wire can be rotated at a high speed. Because of that, the processing can be accomplished with the spindle 13 rotated at a high speed and a good finish of the processing can also be obtained and the cutting resistance acting on the tool 1 can be reduced. Since the spring elements 27A and 27B are disposed between the neighboring rolling bearings 26, the spring elements 27A and 27B can be provided with no need to increase the diameter of the spindle guide section 3.

During the operation of the remote controlled actuator 5 shown in Fig. 1, the respective positions of the tool 1 and the to-be-processed object 6 are estimated by the navigation system and are then displayed on the screen of the display unit 52. Because of this, even when the tool 1 is not visible directly with eyes because the tool 1 is then positioned inside the to-be-processed object 6 such as, for example, the bone, the operator can manipulate the tool 1 while looking at the screen of the display unit 52 to ascertain the position of the tool 1 and the position of the to-be-processed object 6. Also, where the respective positions and attitudes of the actuator main body 10, the distal end member 2, the tool 1 and the to-be-processed object 6 are displayed in the form of the plural dots 60 (as shown in Fig. 9) or the contours thereof are displayed in the form of the graphic symbol 61 (as shown in Fig 10), the position of the tool 1 relative to the to-be-processed object 6 can readily be grasped visually.

In the event that either or both of the tool 1 and the spindle guide section 3 is/are replaced, the experiment shown in Fig. 2A has to be carried out using the actuator mechanism 5a after the replacement so that contents stored in the tool marker relative position and attitude storage section 55 can be rewritten. By so doing, replacement of the tool 1 and/or spindle guide section 3 can be accommodated.

While the actuator mechanism 5a of the remote controlled actuator 5 and the navigation and maneuvering computer 9 are positioned having been spaced a distance from each other, an electrical signal indicative of the attitude of the distal end member 2 detected by the tool attitude detector 45 provided in the actuator mechanism 5a is transmitted to the tool processing member position estimator 55 of the navigation and maneuvering computer 9, shown in Fig. 5, through the actuator electric cable 46 and respective output signals of the tool rotation controller 5ba and the attitude controller 5bb, both built in the navigation and maneuvering computer 9, are transmitted to the tool rotation drive source 41 and the attitude altering drive source 42, both built in the actuator mechanism 5a, through the electric cable 46. Therefore, information transmission between the actuator mechanism 5a and the navigation and maneuvering computer 9 is facilitated.

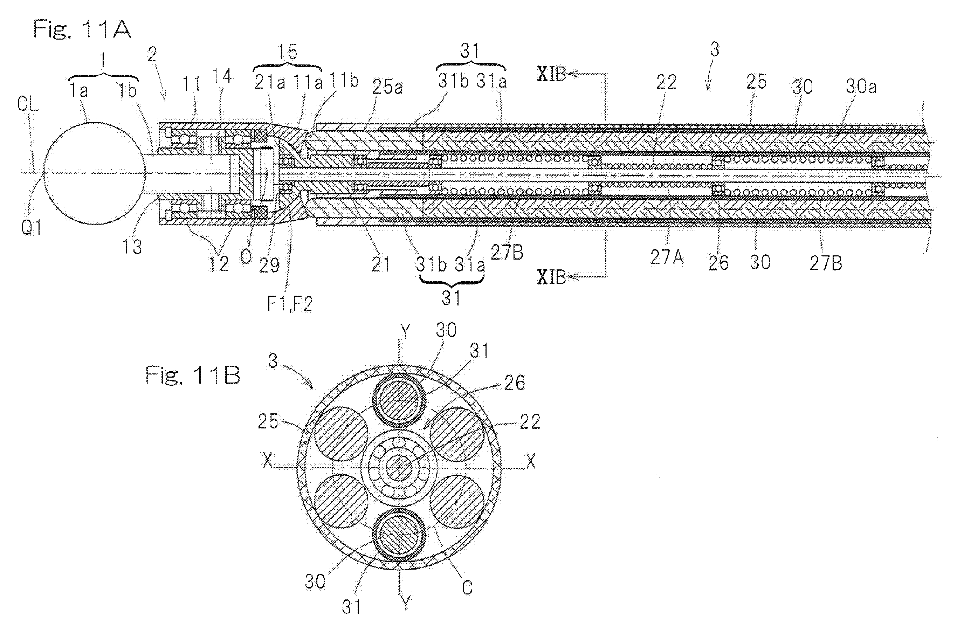

Figs. 11A and 11B illustrate the actuator mechanism 5a of the remote controlled actuator 5 designed in accordance with a second preferred embodiment of the present invention. The spindle guide section 3, which is one of the component parts of the actuator mechanism 5a, is of such a design that as best shown in Fig. 11B, the two guide pipes 30 are provided at the peripheral positions spaced 180° in phase from each other within the outer shell pipe 25 and the attitude altering member 31 is reciprocally movably inserted within guide holes 30a, which are inner diametric holes of the guide pipes 30. Between those two guide pipes 30, a plurality of reinforcement shafts 34 are arranged on the same pitch circle as that of the guide pipes 30. As shown in Fig. 11A, no restoring elastic member 32 (such as best shown in Fig. 3A) is provided. The guide faces F1 and F2 are spherical surfaces each having the center of curvature lying at the point O or cylindrical surfaces each having a lateral X-axis as a longitudinal axis passing through the point O.

The drive unit (corresponding to "4" in Fig. 4A) is provided with two attitude altering drive sources (corresponding to "42" in Fig. 4A) for selectively advancing and retracting respective attitude altering members 31 so that when those two attitude altering drive sources 42 are driven in respective directions opposite to each other, the distal end member 2 can be altered in attitude.

By way of example, when the upper attitude altering member 31 shown in Fig. 11A is advanced towards the tip end side and the lower attitude altering member 31 is retracted, the housing 11 for the distal end member 2 is pressed by the upper attitude altering member 31 and, therefore, the distal end member 2 is altered in attitude along the guide surfaces F1 and F2 with the tip end side oriented downwards as viewed in Fig. 11A. Conversely, when both of the attitude altering members 31 are driven in the directions opposite thereto, the lower attitude altering member 31 urges the housing 11 for the distal end member 2 to allow the distal end member 2 to alter in attitude along the guide surfaces F1 and F2 with the distal end side oriented upwardly as viewed in Fig. 11A. At this time, the pressures from the upper and lower attitude altering members 31 and a reactive force from the constraint member 21 act on the distal end member connecting unit 15 and, accordingly, the attitude of the distal end member 2 is determined in dependence on the balance of those working forces.

According to this construction, since the housing 11 for the distal end member 2 is pressed by the two attitude altering members 31, as compared with the previously described embodiment in which it is pressed by the only attitude altering member 31, the attitude stability of the distal end member 2 can be increased.

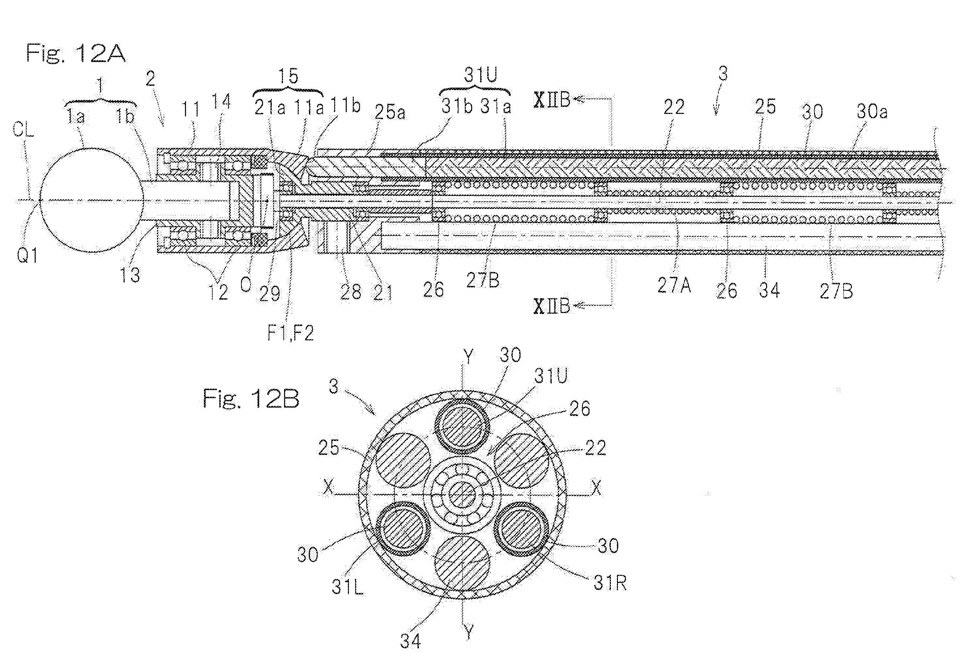

Figs. 12A and 12B illustrate the actuator mechanism 5a employed in the remote controlled actuator 5 designed in accordance with a third preferred embodiment of the present invention. The spindle guide section 3, forming one of the components of the actuator mechanism 5a, is of such a design that as best shown in Fig. 12B, three guide pipes 30 are disposed within the outer shell pipe 25 and positioned at respective circumferential position spaced 120° in phase from each other within the outer shell pipe 25 and, correspondingly, three attitude altering members 31 are accommodated within respective guide holes 30a, which are inner diametric holes of those guide pipes 30, for reciprocal movement relative to the associated guide pipes 30. Between the three guide pipes 30, a plurality of reinforcement shafts 34 are arranged on the same pitch circle as that of the guide pipes 30. As shown in Fig. 12A, no restoring elastic member 32 is provided. The guide surfaces F1 and F2 represents spherical surface having respective centers of curvature lying at the point O and the distal end member 2 can be tilted in any desired direction.

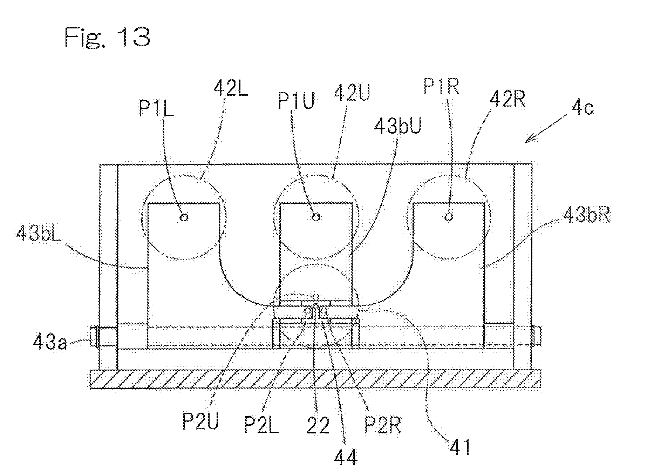

The drive unit is provided with three attitude altering drive sources 42U, 42L and 42R, best shown in Fig. 13, for reciprocally operating respective attitude altering members 31U, 31L and 31R, best shown in Fig. 12B, and those attitude altering drive sources 42 cooperate with each other to drive the distal end member 2 to alter the attitude thereof.

By way of example, when one of the attitude altering members 31U, which is shown in an upper side of Figs. 12A and 12B, is advanced towards the tip end side while the other two attitude altering members 31L and 31R are retracted, the housing 11 for the distal end member 2 is pressed by the attitude altering member 31U shown in the upper side of Figs. 12A and 12B to allow the distal end member 2 to be altered in attitude along the guide surfaces F1 and F2 with the tip end side consequently oriented downwardly as viewed in Fig. 12A. At this time, those attitude altering drive sources 42 are controlled so that the amount of advance or retraction of each of the attitude altering members 31 may become proper. On the other hand, when each of those attitude altering members 31 is conversely retracted or advanced, the housing 11 for the distal end member 2 is pressed by the attitude altering members 31L and 31R, which are shown on lower left and lower right sides, and, consequently, the distal end member 2 is altered in attitude along the guide surfaces F1 and F2 with the tip end side oriented upwardly as viewed in Fig. 12A.

Also, when while the attitude altering member 31U on the upper side as viewed in Fig. 12B is held still, the attitude altering member 31L on the left side is advanced towards the tip end side and the attitude altering member 31R on the right side is retracted, the housing 11 for the distal end member 2, best shown in Fig. 12A, is pressed by the attitude altering member 31L on the left side to allow the distal end member 2 to be oriented rightwards, that is, to be altered in attitude along the guide surfaces F1 and F2 with the distal end member 2 oriented towards a rear side of the sheet of the drawing of Fig. 12A. Conversely, when the attitude altering members 31L and 31R on the left and right sides as viewed in Fig. 12B are advanced and retracted, the housing 11 for the distal end member 2, best shown in Fig. 12A, is pressed by the attitude altering member 31R on the right side, allowing the distal end member 2 to be altered in attitude so that the distal end member 2 can be guided along the guide surfaces F1 and F2 so as to be oriented leftwards.

The use of the attitude altering members 31 at the three circumferential locations as hereinabove described is effective to allow the distal end member 2 to be altered in attitude in two axis directions (X-axis and Y-axis directions) upwardly or downwardly and leftwards or rightwards. At this time, respective pressures from the three attitude altering members 31 and the reactive force from the constraint member 21 act on the distal end member connecting unit 15 and, therefore, the attitude of the distal end member 2 is determined in dependence on the balance of those working forces. According to the above described construction, since the housing 11 for the distal end member 2 is pressed by the three attitude altering members 31, the attitude stability of the distal end member 2 can be further increased. It is, however, to be noted that if the number of the attitude altering members 31 used is increased, the attitude stability of the distal end member 2 can be still further increased.

In the case where the distal end member 2 can be altered in attitude in the two axis directions, the attitude of the distal end member 2 in those two axis directions can be detected by detecting the amount of actuation of at least two of the three attitude altering drive sources 42U, 42L and 42R (Fig. 13) through corresponding actuation amount detectors (not shown). In such case, an aggregation of the actuation amount detectors will form the tool attitude detector 45.

For example, the attitude altering drive mechanism 4c for driving the three attitude altering members 31 is constructed as shown in Fig. 13. In other words, the attitude altering drive mechanism 4c is so constructed that the three attitude altering drive sources 42 (42U, 42L and 42R) for selectively advancing and retracting the attitude altering members 31 (31U, 31L and 31R) may be arranged along a leftward and rightward direction and parallel to each other. Levers 43b (43bU, 43bL and 43bR) corresponding to the attitude altering drive sources 42U, 42L and 42R may be provided for pivotal movement about a common support pin 43a to enable the force of the output rod 42a (42aU, 42aL and 42aR) of each of the attitude altering drive sources 42 to work on the point P1 (P1U, P1L and P1R) of the respective lever 43b, which is spaced a long distance from the support pin 43a, and to enable the force to work on the attitude altering member 31 at the point P2 (P2U, P2L and P2R), which is spaced a short distance from the support pin 43a. Accordingly, the output of each of the attitude altering drive sources 42 can be increased and then transmitted to the corresponding attitude altering member 31. It is to be noted that the rotary shaft 22 is passed through an opening 44 defined in the lever 43bU for the attitude altering member 31U on the upper side.

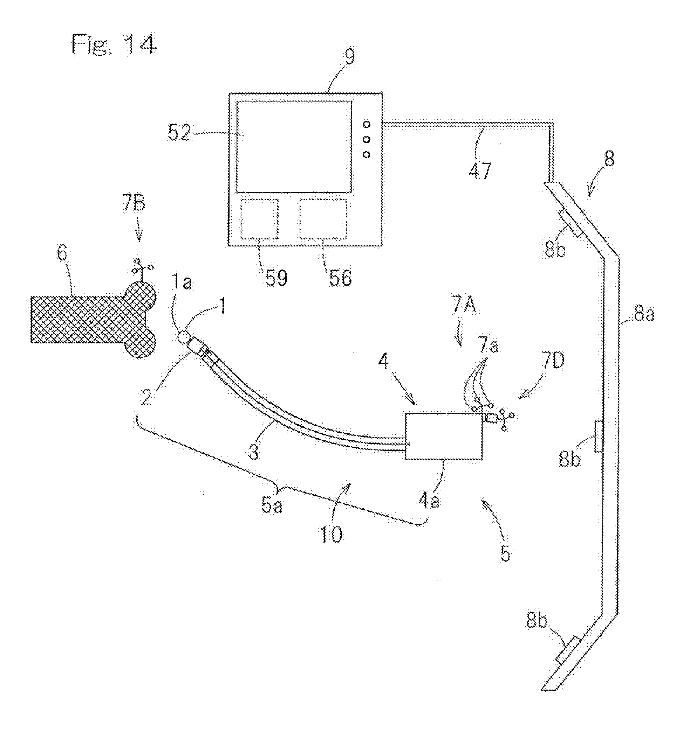

Hereinafter, a mode of application of the present invention will be described with particular reference to Figs. 14 to 25. In the mode of application, components similar to those shown and described in connection with the previously described first embodiments are designated by like reference numerals and, therefore, the details thereof are not reiterated for the sake of brevity. Fig. 14 illustrates a schematic structure of the navigation system for the remote controlled actuator according to this mode of application.

As can readily be understood from Fig. 14, the use of the tool marker 7C, which has been shown and described as essential in the practice of the first embodiment best shown in and described with reference to Fig. 1, is eliminated and a rotational angle detection marker 7D is newly added and, in place of the tool and tool marker relative position storage section 54 and the tool marker relative position and attitude storage section 55, an actuator shape storage section 59 is employed. Also, the tool processing member position estimator 56 is so modified as to differ from the previously described first embodiment of the present invention in that, as will be detailed later, the position of the processing member of the tool can be estimated from various information on the position and attitude of the actuator main body marker, detected by the marker detecting unit, and the position and attitude of the rotational angle detection marker 7D and information stored in the actuator shape storage section 59.

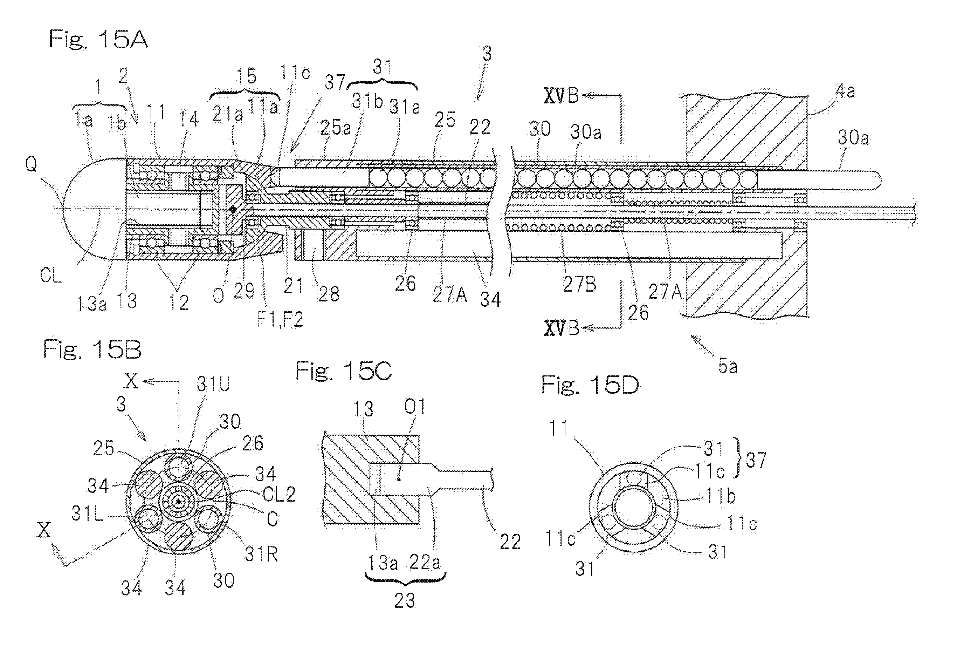

In the case of this mode of application, as shown in Fig. 15B, three guide pipes 30 each having its opposite ends opening, that is, three open-ended guide pipes 30 are provided between the inner diametric surface of the outer shell pipe 25 and the rotary shaft 22 and positioned at respective circumferential locations spaced 120° in phase from each other in the circumferential direction. Within respective guide holes 30a, shown in Fig. 15A, which are inner diametric holes in those guide pipes 30, attitude altering members 31 (31U, 31L and 31R), each of which is comprised of a plurality of balls 31a, forming a force transmitting member, and pillar shaped pins 31b at opposite ends, are accommodated for selective advance and retraction. The balls 31a and the pillar shaped pins 31 are arranged in a row, with no gap formed, in a direction along the lengthwise direction of the respective guide hole 30a. As is the case with the previously described first embodiment, one of the pillar shaped pins 31b adjacent the distal end member 2 has a tip end of a spherical shape and a tip end of that spherical shape is held in engagement with a bottom face of a radial groove 11c formed in the base end face 11b of the housing 11. This radial groove 11c and the pillar shaped pins 31b altogether form a rotation preventing mechanism 37 and, accordingly, when the tip end portion of the pillar shaped pin 31b, then inserted into the radial groove 11c, contacts a side face of the radial groove 11c, the distal end member 2 shown in Fig. 15A can be prevented from rotating about the center line C of the distal end member 2 relative to the spindle guide section 3. Even the other of the pillar shaped pins 31b adjacent the drive unit housing 4a has a spherical tip end and this spherical tip end is held in engagement with a side face of the pivot lever 43b.

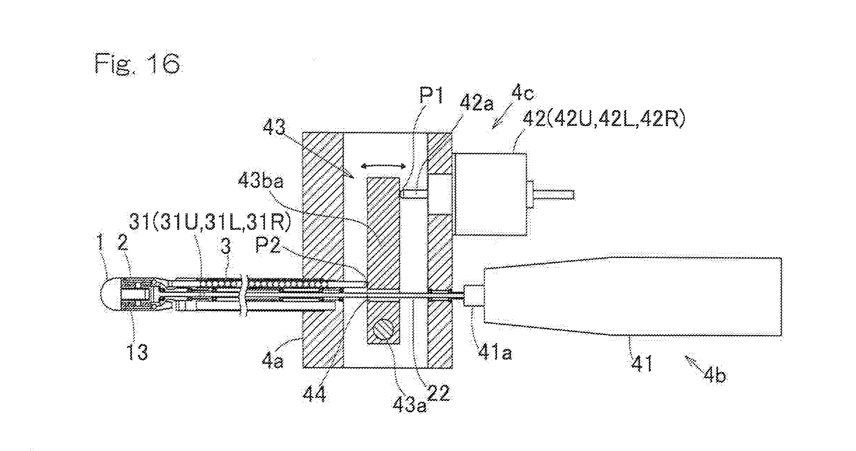

As shown in Fig. 16, the attitude altering drive mechanism 4c includes three attitude altering drive sources 42 (42U, 42L and 42R) corresponding to the attitude altering members 31 (31U, 31L and 31R). Each of the attitude altering drive sources 42 is in the form of, for example, an electrically operated linear actuator and an output rod 42a thereof, which moves a leftward and rightward direction as viewed in Fig. 16, is transmitted to the corresponding attitude altering member 31 through the lever mechanism 43. Each of the attitude altering drive sources 42 may, however, be employed in the form of a rotary motor.

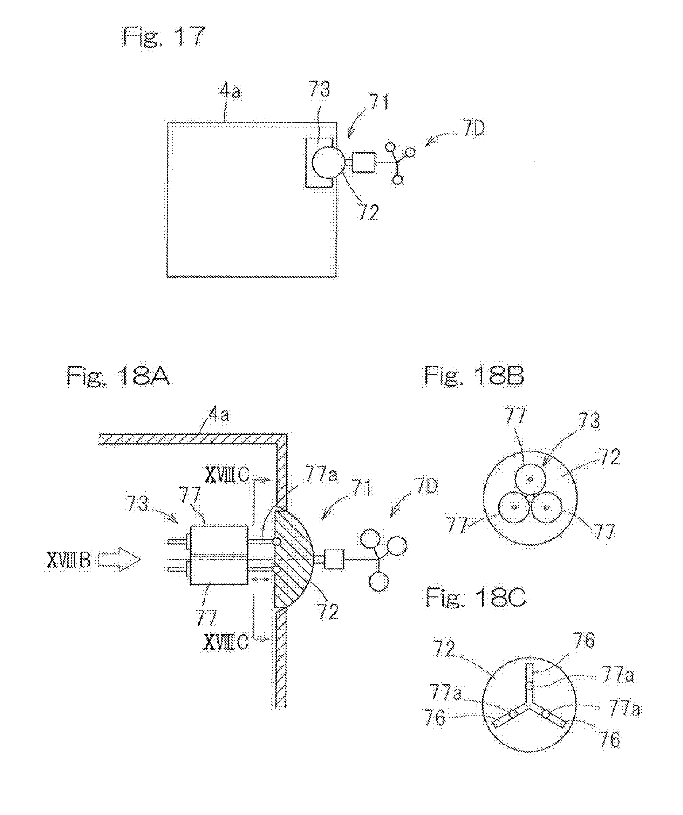

The marker detecting unit 8 shown in Fig. 14 makes use of the markers 7A, 7B and 7D as respective object to be detected. The actuator main body marker 7A is fixedly fitted to the drive unit housing 4a, which forms a part of the actuator main body 10, and the object marker 7B is fixedly fitted to the to-be-processed object 6 such as, for example, the bone or the like. The rotational angle detection marker 7D is provided in the drive unit housing 4a through a marker support mechanism 71, for example, as shown in Fig. 17. The marker support mechanism 71 includes a spherical marker support member 72 rotatable in any arbitrary direction along a spherical surface and is operable to rotationally drive the marker support member 72 in the arbitrary direction by means of a marker drive actuator 73. The rotational angle detection marker 7D is supported by the marker support member 72. The marker drive actuator 73 operates in operative association with the pivotal movement of the distal end member 2 about the pivot center O to thereby permit the rotational angle detection marker 7D to alter in attitude in the same direction and through the same angle as those of, for example, the distal end member 2. If the relation between the angular displacement of the distal end member 2 and the angular displacement of the marker 7D is fixed at all times, the marker 7D may be altered in attitude in a direction counter to the distal end member 2. For the marker drive actuator 73, a spherical actuator of a type utilizing a surface wave type ultrasonic vibration and having the freedom in three directions perpendicular to each other can be employed. For the details of the spherical actuator utilizing the surface wave type ultrasonic vibration, reference may be made to the JP Laid-open Patent Publication No. S62-228392.

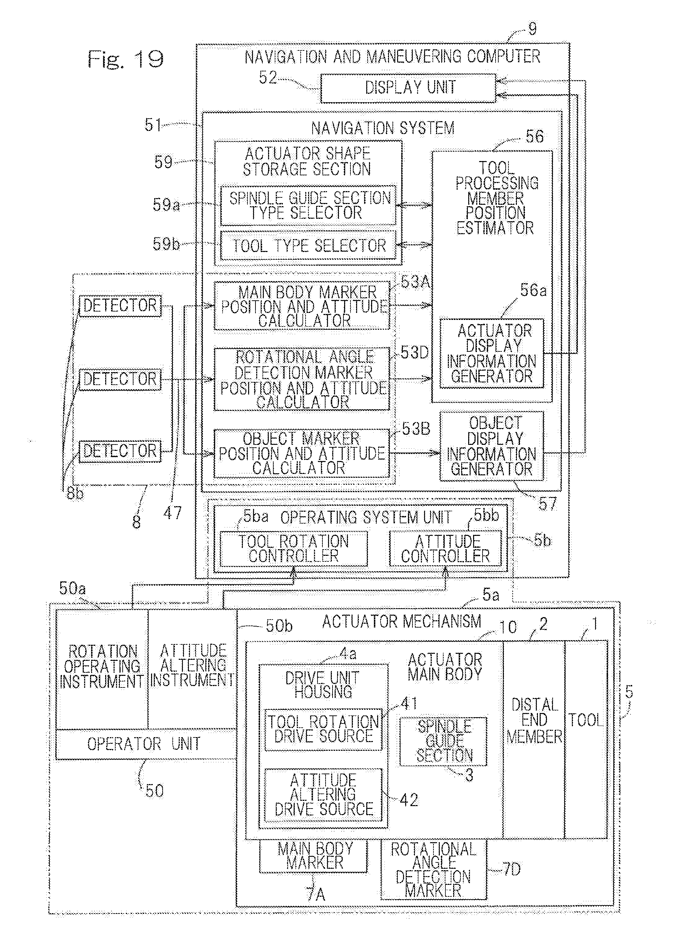

The operative association of the marker drive actuator 73 with the distal end member 2 is carried through, for example, an operative association control mechanism (not shown) capable of outputting to the marker drive actuator 73, shown in Fig. 17, a command synchronized with a command fed from the attitude altering instrument 50b, shown in Fig. 19, to the attitude altering drive source 42. This operative association control mechanism is provided within, for example, the drive unit housing 4a.

The marker support mechanism 71 may be constructed as shown in Figs. 18A to 18C. In other words, as shown in Fig. 18A, the marker support member 72 of the marker support mechanism 71 represents a semispherical shape and the rotational angle detection marker 7D is supported on a spherical surface portion thereof. A flat surface portion of the marker support member 72 is formed with three directional guide grooves 76, best shown in Fig. 18C, and respective tip ends of output shafts 77a of three linear motors 77, best shown in Fig. 18B, forming the marker drive actuator 73 are engaged in those guide grooves 76. When those linear motors 77 are driven in operative association with the pivotal movement of the distal end member 2 about the pivot center O, the attitude of the rotational angle detection marker 7D can be altered.

Even in this case, in a manner similar to the marker support mechanism 71 shown in and described with reference to Fig. 17, control is so made that the pivot of the marker support member 72, best shown in Figs. 18A to 18C, about its pivot center may take place in the same direction (or in a direction counter to) and over the same pivotal angle as those of the distal end member 2 (best shown in Fig. 14) about the pivot center O. More specifically, based on information from the attitude detector (not shown) for the distal end member 2, the amount of movement of each of the linear motors 77 is calculated by a linear motor controller (not shown) and each of those linear motors 77 is then driven by the amount of movement so calculated.

The marker detecting unit 8 shown in Fig. 14 includes three individual detectors 8b supported by the detector support body 8a, and marker position and attitude calculators 53A, 53B and 53D built in the navigation and maneuvering computer 9 best shown in Fig. 19. In association with the individual detectors 8b shown in Fig. 14, each of the markers 7A, 7B and 7D is provided with three light reflectors 7a. Those three light reflectors 7a are held at different positions, respectively. The individual detectors 8b are of an optical type as is the case with those employed in the practice of the previously described first embodiment and are operable to project respective detection light towards the light reflectors 7a of the markers 7A, 7B and 7D and then to receive the detection light reflected from the associated markers 7A, 7B and 7D. The detection signals of those individual detectors 8b are fed respectively to the marker position and attitude calculators 53A, 53B and 53D, best shown in Fig. 19 and all built in the navigation and maneuvering computer 9, through a wiring system (not shown), built in the detector support body 8a, and a marker detector electric cable 47. It is, however, to be noted that the provision may be made of three light projectors (not shown) in the markers 7A, 7B and 7D such that detection light projected from those light projectors can be received by the individual detectors 8b.

The navigation system 51 includes the actuator shape storage section 59, marker position and attitude calculators 53A, 53B and 53D for the main body actuator, the rotational angle detection and the object to be processed, and a tool processing member position estimator 56. The actuator shape storage section 59 includes a spindle guide section type selector 59a and a tool type selector 59b. The tool processing member position estimator 56 includes an actuator display information generator 56a. Also, separate therefrom, the navigation system 51 also includes an object display information generator 57.

The actuator shape storage section 59 is used to store information on the relative position of the pivot center O, about which the distal end member 2 pivots, relative to the actuator main body marker 7A fitted to the drive unit housing 4a best shown in Fig. 15A, and information on the shape of the tool 1 with the pivot center O taken as a reference. For the information on the shape of the tool 1, for example, information on the relative position of the tip end Q (best shown in Fig. 2B) of the processing member 1a relative to the pivot center O when the attitude of the distal end member 2 is held in a neutral position. The information on this relative position may be merely a distance between the pivot center O of the distal end member 2 and the tip end Q of the processing member 1a.

The actuator shape storage section 59 will now be further described in detail.

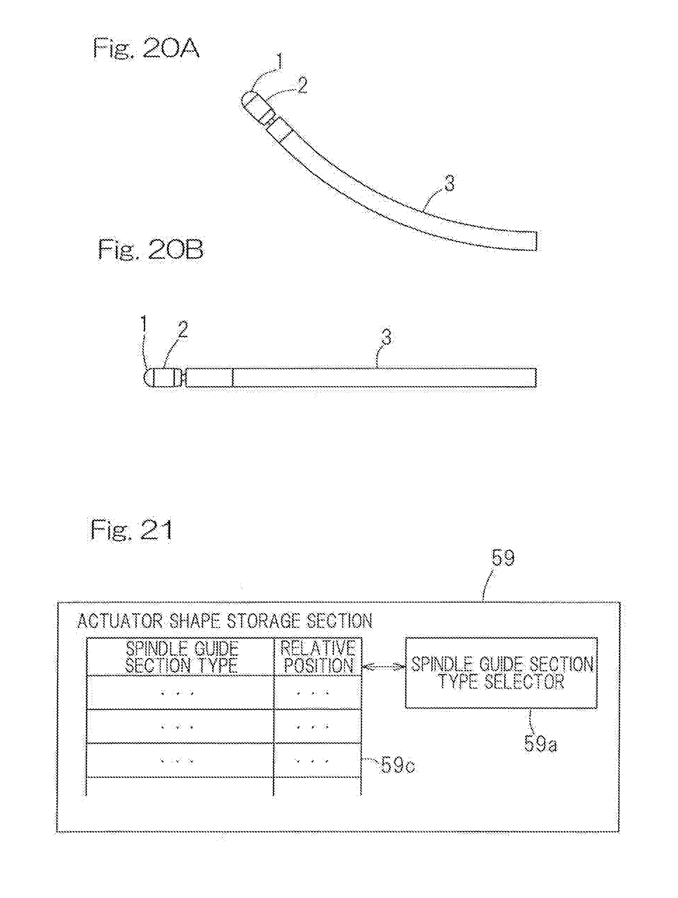

The relative position of the pivot center O of the distal end member 2 relative to the actuator main body marker 7A depends on the shape of the spindle guide section 3. For example, the relative position referred to above differs depending on whether the spindle guide section 3 is of a curved shape as shown in Fig. 20A or whether the spindle guide section 3 is of a linear shape. Even where the spindle guide section 3 is artificially deformed, the relative position referred to above varies depending on whether it is before the deformation or whether it is after the deformation. The actuator shape storage section 59 has a table 59c accommodated therein, which table 59c stores relations between types of spindle guide sections 3 and the associated relative positions, and the spindle guide section type selector 59a selects a proper relation out from the plural relations memorized and stored in this table 59c depending on the information inputted from the outside. The relative position for each of the types of the spindle guide sections 3 is determined beforehand by means of measurements or design data.

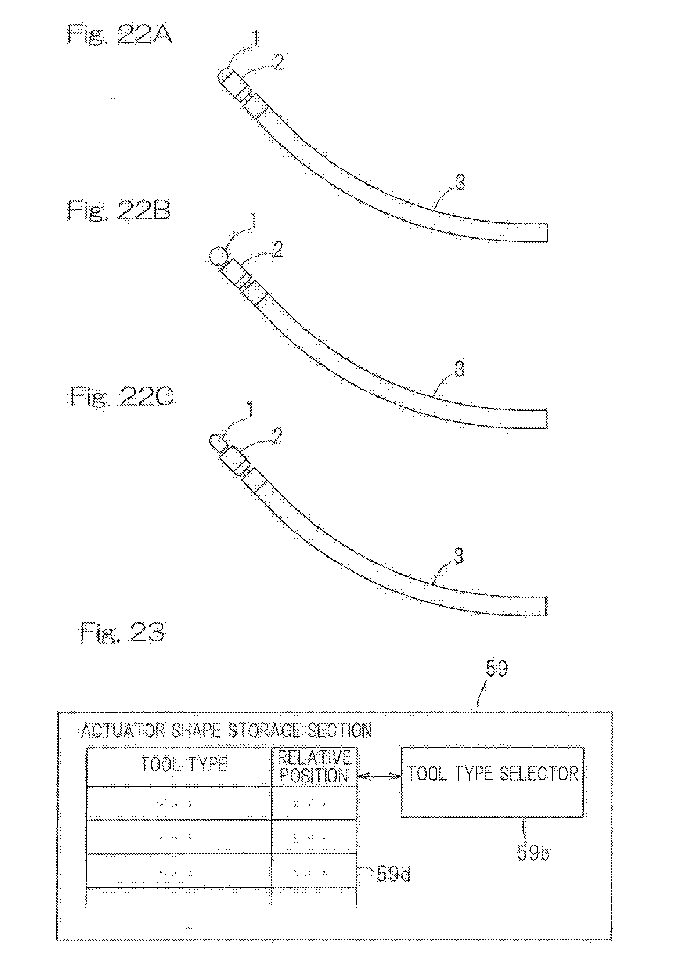

The shape of the tool 1 using the pivot center O as a reference varies depending on the type of the tool 1 used. For example, the relative position referred to above differs depending on whether the tool 1 of a type having the semispherical processing member 1a as shown in Fig. 22A is used, whether the tool 1 of a type having the spherical processing member 1a as shown in Fig 22B is used, or whether the tool 1 of a type having the pillar shaped processing member 1a as shown in Fig. 22C is used. The actuator shape storage section 59 has a table 59d accommodated therein, which table 59d memorizes and stores relations between the types of the tool 1 and the associated relative positions, and the tool type selector 59b selects a proper relation out from the plural relations memorized and stored in the table 59d depending on the information inputted from the outside. The relative positions of the tools 1 of those types are determined beforehand by means of measurements or design data.

The marker position and attitude calculators 53A, 53B and 53D calculate respective positions and attitudes of the respective markers 7A, 7B and 7D from the detection signals of the three detectors 8b of the marker detecting unit 8. When the light reflectors 7a of the markers 7A, 7B and 7D and the detectors 8b of the marker detecting unit 8 are employed each in three or more in number, respective three dimensional positions and attitudes of the markers 7A, 7B and 7C can be determined. The position and attitude of the actuator main body marker 7A are synonymous to the position and attitude of the actuator main body 10. In other words, by the marker position and attitude calculator 53A, the position and the attitude of a reference portion of the actuator main body 10 can be detected. The term "reference portion" referred to above and hereinafter is intended to mean a portion that provides a reference for the calculation performed by a tool processing member position estimator 56 as will be described later. The position and attitude of the object marker 7B are synonymous to the position and attitude of the to-be-processed object 6. The marker position and attitude calculator 53D are operable to calculate the angle of rotation of the distal end member 2 from both of the attitude of the rotational angle detection mark 7D and the position and attitude of the actuator main body 10.

The tool processing member position estimator 56 referred to above estimates the position of the tip end Q of the processing member 1a of the tool 1 from the information on the position and attitude of the actuator main body marker 7A, detected by the marker detecting unit 8 shown in Fig. 14 and the information on the attitude of the rotational angle detection mark 7D, as well as the information stored in the actuator shape storage section 59.

In other words, the tool processing member position estimator 56 can estimate the absolute position of the pivot center O from the information on the position and attitude of the actuator main body marker 7A detected by the marker detecting unit 8 shown in Fig. 14, that is, the position and attitude of the reference portion of the actuator main body 10, and the information on the relative position of the pivot center O of the distal end member 2, shown in Fig. 15A, relative to the marker 7A stored in the actuator shape storage section 59. Also, the relative position of the distal end Q of the processing member 1a of the tool 1 relative to the pivot center O can be estimated from the information on the attitude of the rotational angle detection marker 7D detected by the marker detecting unit 8, that is, the information on the angle of rotation of the distal end member 2 about the pivot center O, and the information on the shape of the tool 1 stored in the actuator shape storage section 59.

The absolute position of the tip end Q of the processing member 1a of the tool 1 can be estimated from the absolute position of the pivot center O of the distal end member 2 so estimated in the manner described above and the relative position of the tip end Q of the processing member 1a of the tool 1 with reference to the pivot center O. For this reason, the position of the processing member 1a of the tool can be estimated relative to the remote controlled actuator 5 of the type capable of altering the position of the distal end member 2 for the support of the tool provided in the distal end portion of the spindle guide section 3.

The actuator display information generator 56a shown in Fig. 19 calculates the actuator display information, which is the information capable of displaying the position and attitude of the actuator main body 10, and the attitude of the distal end member 2, from the input information used in estimating the position of the processing member 1a of the tool 1 shown in Fig. 14, and then displays the result of such calculation on the screen of the display unit 52. Also, the object display information generator 57 calculates the object display information, which is the information on the position and attitude of the object marker 7B determined by the marker position and attitude calculator 54C, and then displays the result of such calculation on the screen of the display unit 52.

More specifically, as best shown in Fig. 9, the actuator display information and the object display information, that is, the position and attitude of the actuator main body 10, the attitude of the distal end member 2 and the position and attitude of the to-be-processed object 6 is displayed in the form of the series of the dots 60. Alternatively, as shown in Fig. 10, using a computer graphics or the like, based on the shape information on various parts of the actuator main body 10 registered in the navigation maneuvering computer 9, respective external shapes of the to-be-processed object 6, the tool 1, the distal end member 2 and the spindle guide section 3, the respective positions and attitudes of which have been reflected, are displayed in the form of graphic symbols. Also, as shown in Figs. 9 and 10, together with the display in the form of the dots 60 and the graphic symbols 61, the actuator display information and the object display information may be displayed in the form of numerals on the screen of the display windows 62.

The operation of the remote controlled actuator 5 of the structure shown in and described with particular reference to Fig. 14 will be described hereinafter.

Assuming that the tool rotation drive source 41 shown in Fig. 16 is driven, the rotational force thereof is transmitted to the spindle 13 through the rotary shaft 22 so as to rotate the tool 1 together with the spindle 13. By the tool 1 so rotated, the bone or the like is cut. The rotational speed of the tool 1 can be set arbitrarily to any desired value by means of the rotation operating instrument 50a best shown in Fig. 19.

During the use, through the manipulation of the attitude altering instrument 50b, the attitude altering drive sources 42 (42U, 42L and 42R) best shown in Fig. 16 are drive in operative association with each other to advance and retract the attitude altering members 31 (31U, 31L and 31R) so as to alter the attitude of the distal end member 2.

For example, the upper attitude altering member 31U shown in Fig. 15B is advanced towards the tip end side and the remaining two attitude altering members 31L and 31R are retracted. Once this takes place, the housing 11 for the distal end member 2 is pressed by the upper attitude altering member 31U with the distal end member 2 consequently altered in attitude along the guide faces F1 and F2 so as to permit the tip end side thereof to be oriented downwards in Fig. 15A. At this time, each of the attitude altering drive sources 42 (shown in Fig. 16) is controlled so that the amount of advance or retraction of the corresponding attitude altering member 31 can attain a proper value. In the event that each of the attitude altering members 31 is advanced or retracted in a direction reverse to that described above, the housing 11 for the distal end member 2 is pressed by the left and right attitude altering members 31L and 31R with the distal end member 2 consequently altered in attitude along the guide faces F1 and F2 so as to permit the tip end side to be oriented upwardly in Fig. 15A.

Also, when, while the upper attitude altering member 31U as viewed in Fig. 15B is held standstill, the left attitude altering member 31L is caused to advance towards the tip end side and the right attitude altering member 31R is caused to retract, the housing 11 of the distal end member 2, as viewed in Fig. 15A, is pressed by the left attitude altering member 31L and, consequently, the distal end member 2 is altered in attitude so as to be oriented rightwards, that is, towards the side rearwardly of the plane of the sheet of Fig. 15A, along the guide faces F1 and F2. On the other hand, when the attitude altering members 31L and 31R are advanced or retracted in a manner reverse to those above mentioned, the housing 11 of the distal end member 2 is therefore pressed by the right attitude altering member 31R, resulting in the attitude of the distal end member 2 altered so as to be oriented leftwards along the guide faces F1 and F2.

Since this embodiment makes use of the rotation preventing mechanism 37 (Fig. 15D) for preventing the distal end member 2 from rotating about the center line CL of the distal end member 2 relative to the spindle guide section 3, even when the distal end member 2 then holding the tool 1 becomes uncontrollable as a result of any trouble occurring in the attitude altering drive mechanism 4c (shown in Fig. 16) for controlling the selective advance or retraction of the attitude altering member 31 and any other control devices, it is possible to avoid the possibility that the to-be-processed object 6 may be impaired as a result of rotation of the distal end member 2 about the center line CL 1 or the distal end member 2 itself is broken.

Since the attitude altering member 31 is inserted through the guide hole 30a in the guide pipe 30, the attitude altering member 31 can properly act on the distal end member 2 at all times without being accompanied by displacement in position in a direction perpendicular to the lengthwise direction thereof and the attitude altering operation of the distal end member 2 can therefore be performed accurately. Also, since the attitude altering member 31 is made up of the plural balls 31c and the pillar shaped pins 31b and has a flexibly property in its entirety, the attitude altering operation of the distal end member 2 is carried out accurately even though the spindle guide section 3 is curved. In addition, since the center of the junction between the spindle 13 and the rotary shaft 22 lies at the same position as the pivot center O, no force tending to press and pull will not act on the rotary shaft 22 as a result of the alteration of the attitude of the distal end member 2 and the distal end member 2 can be smoothly altered in attitude.

In the event that the spindle guide section 3 is replaced with a different type of the spindle guide section or the spindle guide section 3 is deformed with the spindle section of a different shape, such replacement or deformation can be accommodated since the spindle guide section type selector 59a selects a proper relation out from the relations between the types of the spindle guide section 3 and the relative position of the distal end member 2, which are stored in the table 59c in the actuator shape storage section 59 shown in Fig. 19. Similarly, even when the tool 1 is replaced with a type having a different shape, such replacement can be accommodated since the tool type selector 53b selects a proper relation out from the relations between the types of the tools 1 and the relative positions of the processing member 1a, which are stored in the table 59d in the actuator shape storage section 59.

In the control of the navigation for estimating the position of the tool 1 shown in Fig. 14, the position and attitude of the reference portion of the actuator main body 10 and the angle of rotation of the distal end member 2 about the pivot center O are detected by the marker detecting unit 8. In other words, the information relating to the actuator mechanism 5a in the remote controlled actuator 5, which is one of pieces of the information necessary to estimate the position of the processing member 1a of the tool 1, can be detected at a site distant from the actuator mechanism 5a. For this reason, there is no necessity that the actuator mechanism 5a of the remote controlled actuator 5 and the navigation and maneuvering computer 9 necessary to control it are connected with each other through a cable, and, hence, a good handlability of the actuator mechanism 5a can be appreciated.

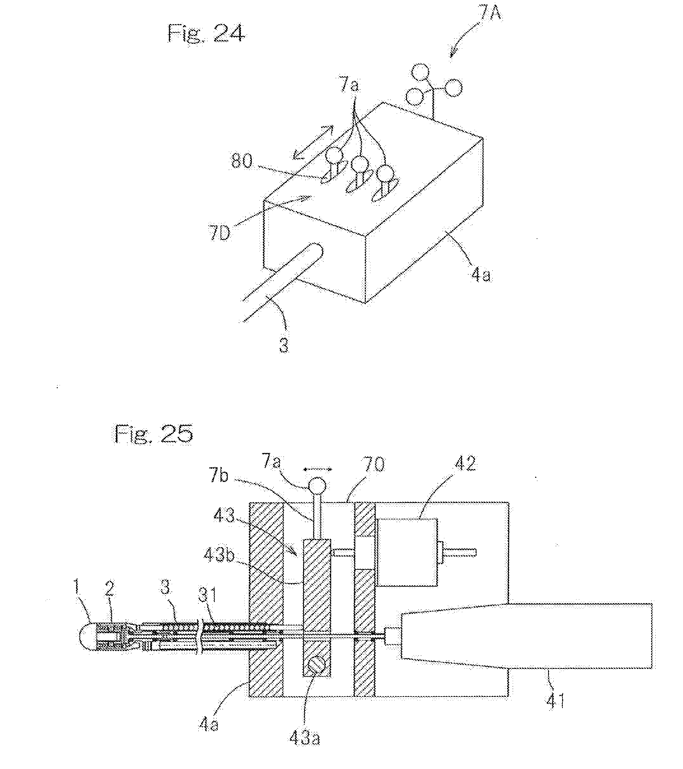

Figs. 24 and 25 illustrate a drive unit housing 4a used in association with the different navigation system. This navigation system is so designed and so configured that the angle of rotation of the distal end member 2, shown in Fig. 15A, about the pivot center O can be detected by detecting the position of the rotational angle detection marker 7D by means of the marker detecting unit 8. In the case of this navigation system, the three light reflectors 7a of the rotational angle detection marker 7D are independent from each other and each of those light reflectors 7a is fitted to the pivot lever 43b of the respective lever mechanism 43 between the attitude altering drive source 42 (42U, 42L and 42R), shown in Fig. 16, and the associated attitude altering member 31 (31U, 31L and 31R) through the support member 7b extending through an opening 80 defined in the drive unit housing 4a. Each of the light reflectors 7a is detected as to its position by the corresponding detector 8b in the marker detecting unit 8 shown in Fig. 14.

Since, at the time the distal end member 2 shown in Fig. 16 changes its attitude, the pivot levers 43b pivot independent of each other, detection of the respective positions of the light reflectors 7a of the rotational angle detection marker 7D shown in Fig. 24 by means of the marker detecting unit 8 makes it possible to grasp the amount of pivot of each of those pivot levers 43b shown in Fig. 16 and from the amount of pivot the angle of rotation of the distal end member 2 shown in Fig. 15A about the pivot center O can be estimated. The tool processing member position estimator 56 shown in Fig. 14 estimates the position of the tip end Q of the processing member 1a of the tool 1 from the rotational angle so obtained, the position and attitude of the actuator main body marker 7A detected by the marker detecting unit 8 and the information stored in the actuator shape storage section 59.

As hereinabove described, since the use of the rotational angle detection marker 7D in the transmission system through which the operation is transmitted from the attitude altering drive source 42 shown in Fig. 16 to the distal end member 2 is effective to eliminate the use of any extra member for detecting the position of the rotational angle detection marker 7D, the structure can be simplified. It is to be noted that the rotational angle detection marker 7D may be provided in an operating portion of the attitude altering drive source 42, for example, in the output rod 42 thereof.

Although in describing the navigation system for the remote controlled actuator reference has been made to that for the medical use, the present invention can be equally applied to the navigation system for the remote controlled actuator for use in any application. By way of example, if the remote controlled actuator is used in performing a mechanical processing, a drilling process for drilling a curved hole and a cutting process to be performed at a site deep in the groove can be accomplished.

The mode of application described hereinbefore includes the following modes of application, all of which do not require the use of the tool marker, the tool and tool marker relative position storage section and the tool marker relative position and attitude storage section employed in the practice of any one of the previously described first to third embodiments.

### [Mode 1]

The navigation system for the remote controlled actuator according to this mode 1 is such that in the navigation system for estimating the position of the processing member 1a of the tool 1 relative to the remote controlled actuator 5, which actuator includes the actuator main body 10, in which the base end of the spindle guide section 3 of the elongated configuration is coupled with the drive unit housing 4a; the distal end member 2 fitted to the distal end portion of the spindle guide section 3 for pivotal movement about the pivot center O to enable it to be altered in attitude; the tool 1 rotatably supported by the distal end member 2; an attitude altering drive source 42 and a tool rotation drive source 41 both provided within the drive unit housing 4a for altering the attitude of the distal end member 2 and rotating the tool 1, respectively; and an operator unit 50 provided in the drive unit housing 4a for controlling respective operations of the drive sources 42 and 41 for maneuvering the attitude of the distal end member 2 and the rotation of the tool 1, there is provided the marker detecting unit 8 for detecting the position and attitude of the actuator main body marker 7A, fitted to the drive unit housing 4a of the actuator main body 10, and the position and attitude of the rotational angle detection marker 7D operable in operative association with the pivot movement of the distal end member 2 about the pivot center O; the actuator shape storage section 59 for storing the information on the relative position of the pivot center O relative to the actuator main body marker 7A and the information on the shape of the tool 1 with reference to the pivot center O; and the tool processing member position estimator 56 for estimating the position of the processing member 1a of the tool 1 from the information on the position and attitude of the actuator main body marker 7A, detected by the marker detecting unit 8, the information on the position and attitude of the rotational angle detection marker 7D and the information stored in the actuator shape storage section 59.

According to the above described construction, the marker detecting unit 8 detects the position and attitude of the actuator main body marker 7A, fitted to the drive unit housing 4a of the actuator main body 10, and the position and attitude of the rotational angle detection marker 7D operable in operative association with the pivotal movement of the distal end member 2 about the pivot center O. From the position and attitude of the actuator main body marker 7A, the position and attitude of the reference portion of the actuator main body 10 is detected. Also, from the relation between the position and attitude of the rotational angle detection marker 7D and the position and attitude of the reference portion of the actuator main body 10 acquired in advance, the angle of rotation of the distal end member 2 about the pivot center O is determined.

The tool processing member position estimator 56 estimates the position of the processing member 1a of the tool 1 from the information on the position and attitude of the actuator main body marker 7A, detected by the marker detecting unit 8, the information on the position and attitude of the rotational angle detection marker 7D and the information stored in the actuator shape storage section 59. In other words, the tool processing member position estimator 56 can estimate the absolute position of the pivot center O from the information on the position and attitude of the actuator main body marker 7A detected by the marker detecting unit 8, that is, the information on the position and attitude of the reference portion of the actuator main body 10, and the information on the relative position of the center of pivot O of the distal end member 2 relative to the actuator main body marker 7A stored in the actuator shape storage section 59. Also, the relative position of the processing member 1a of the tool 1 relative to the pivot center O can be estimated from the information on the position and attitude of the rotational angle detection marker 7D detected by the marker detecting unit 8, that is, the information on the angle of rotation of the distal end member 2 about the pivot center O and the information on the shape of the tool 1 stored in the actuator shape storage section 59.

From the absolute position of the center of pivot O of the distal end member 2, estimated in the manner described above, and the relative position of the processing member 1a of the tool 1 with reference to the position of the pivot center O, the absolute position of the processing member 1a of the tool 1 can be estimated. For this reason, relative to the remote controlled actuator 5 of the type capable of altering the attitude of the distal end member 2 for the support of the tool, which is provided in the distal end portion of the spindle guide section 3, the position of the processing member 1a of the tool 1 can be estimated.

As hereinabove described, the position and attitude of the reference portion of the actuator main body 10 and the angle of rotation of the distal end member 2 about the pivot center O can be detected by the marker detecting unit 8. In other words, of the information necessary to estimate the position of the processing member 1a of the tool 1, the information concerning the actuator mechanism 5a in the remote controlled actuator 5 can be detected at the site distant from the actuator mechanism 5a. For this reason, where the actuator mechanism 5a of the remote controlled actuator 5 and the computer 9 used to control the actuator mechanism 5a are separated from each other, there is no need to connect the both by means of the cable and a good handlability of the actuator mechanism 5a can be appreciated. In particular, where the actuator main body 10 including the control system is provided with a compact battery and can therefore operate standalone, the foregoing advantages can be effectively obtained. Also, the information concerning the actuator mechanism 5a can be transmitted wireless to the computer 9, but in such case, it may be adversely affected by communication troubles. If the markers 7A and 7D and the marker detecting unit 8 are employed such as in this mode 1, nothing will be hardly affected by the communication troubles.

### [Mode 2]

In the mode 1 described above, where the marker detecting unit 8 is designed to detect the attitude of the rotational angle detection marker 7D, it is recommended to alter the attitude of the rotational angle detection marker 7D by means of a marker drive actuator operable in operative association with the pivotal movement of the distal end member 2 about the pivot center O.

When the marker drive actuator 73 is used, the attitude of the rotational angle detection marker 7D can be assuredly altered in operative association with the pivotal movement of the distal end member 2 about the pivot center thereof.

### [Mode 3]

By way of example, the marker drive actuator 73 can be employed in the form of a spherical actuator of a type utilizing the surface wave type ultrasonic vibration and having a freedom of three directions perpendicular to each other. Regarding the details of the spherical actuator utilizing the surface wave type ultrasonic vibration, reference may be made to the JP Laid-open Patent Publication No. S62-228392 referred to previously.

### [Mode 4]

Alternatively, the marker drive actuator 73 may be comprised of three linear motors 77 such that by those linear motors 77, the angle of the marker support member 72 supporting the rotational angle detection marker 7D can be changed.

### [Mode 5]

In the mode 1 described above, where the marker detecting unit 8 is of the type capable of detecting the position of the rotational angle detection marker 7D, the rotational angle detection marker 7D may be provided in the attitude altering drive source 42, or the transmission system for transmitting the operation from the attitude altering drive source 42 to the distal end member 2, so that the position thereof can change in operative association with this transmission system.

If the rotational angle detection marker 7D is provided in the above described transmission system, there is no need to employ any extra member for detecting the position of the rotational angle detection marker 7D and, therefore, the structure can be simplified.

### [Mode 6]

In the mode 1 described above, in order for some types of remote controlled actuators in which types of the spindle guide sections 3 are different from each other, to be useable, the actuator shape storage section 59 stores for each of the types of the spindle guide sections 3, the information on the relative position of the pivot point O relative to the actuator main body marker 7A, and the use may be of the spindle guide section type selector 59a for selecting the information of the relative position of the pivot center O that is to be used in estimation by the tool processing member position estimator 56.

Where a certain remote controlled actuator 5 is to be used, from the information on the relative position of the pivot center O relative to the actuator main body marker 7A stored in the actuator shape storage section 59, the information corresponding to the type of the spindle guide section 3 in the remote controlled actuator 5 to be used is selected by the spindle guide section type selector 59a. Accordingly, the navigation system can be useable to the types of the remote controlled actuators 5 utilizing the respective spindle guide sections 3 of the different types.

### [Mode 7]

In the mode 1 described above, in order to enable the plural types of the remote controlled actuators 5, employing the tools 1 of different types, and the remote controlled actuator 5 capable of replacing one of the different types of the tools 1 with another type to be useable, the actuator shape storage section 59 stores for each of the types of the tools 1, the information on the shape of the tool 1 with reference to the pivot center O and the use is preferred of the tool type selector 59b for selecting the information on the shape of the tool 1 that is used in estimation by the tool processing member position estimator 56.

Where a certain remote controlled actuator 5 is to be used, from the information stored in the actuator shape storage section 59 on the shape of the tool 1 with reference to the pivot center O, the information corresponding to the tool 1 in the remote controlled actuator 5 is selected by the tool type selector 59b. Accordingly, the navigation system can be useable to the types of the remote controlled actuators 5 utilizing the respective tool 1 of the different types.

### [Mode 8]

In the mode 1 described above, each of the actuator main body marker 7A and the rotational angle detection marker 7D is of a type capable of projecting or reflecting light and the marker detecting unit 8 can be of an optical type capable of receiving light from the actuator main body marker 7A and the rotational angle detection marker 7D. The optical type marker detecting unit 8 has a simplified structure.

### [Mode 9]

In the mode described above, the use is made of the display unit 52 for displaying images on the screen and the provision of the actuator display information generator 55a in the tool processing member position estimator 56 is preferred so that, from various input information that is used in estimating the position of the tool 1, the actuator display information, which is the information for displaying the position and attitude of the actuator main body 10 and the attitude of the distal end member 2, can be calculated and then displayed on the screen of the display unit 52.

### [Mode 10]

In the mode 9 described above, where the display unit 52 and the actuator display information generator 55a are employed, the actuator display information generated by the actuator display information generator 55a may be the information necessary to display the attitude of the distal end member and the position and attitude of the actuator main body 10 on the screen of the display unit 52 in the form of the series of the dots 60.

### [Mode 11]

In the mode 9 described above, the actuator display information generator 56a may be of a type generating, as the actuator display information, the graphic symbol 61 and then displaying the graphic symbol 61 on the screen of the display unit 52, which symbol 61 is representative of the external shape of the tool 1, the distal end member 2 and the actuator main body 10, which reflect the position and attitude of them, by means of the computer graphics.

### [Mode 12]

In the mode 9 described above, the actuator display information generated by the actuator display information generator 55a may further be the information in the form of the numerals on the screen of the display unit 52.

### [Mode 13]

In the mode 1 described above, the remote controlled actuator is so preferred as to be constructed as follows. In other words, the distal end member 2 is designed to rotatably support the spindle 13 for holding the tool 1; the spindle guide section 3 includes the rotatable shaft 22 for transmitting the rotation of the tool rotation drive source 41 within the drive unit housing 4a to the spindle 13 and the attitude altering member 31 for altering the attitude of the distal end member 2 by selectively advancing or retracting by means of the attitude altering drive source 42 within the drive unit housing 4a in a condition with the tip end held in contact with the distal end member 2.

Although the present invention has been fully described in connection with the preferred embodiments thereof with reference to the accompanying drawings which are used only for the purpose of illustration, those skilled in the art will readily conceive numerous changes and modifications within the framework of obviousness upon the reading of the specification herein presented of the present invention. Accordingly, such changes and modifications are, unless they depart from the scope of the present invention as delivered from the claims annexed hereto, to be construed as included therein.

### [Reference Numerals]

- 1: Tool
- 1a: Processing member
- 2: Distal end member
- 3: Spindle guide section
- 4a: Drive unit housing
- 5: Remote controlled actuator
- 6: Object to be processed
- 7A: Main body marker
- 7B: Object marker
- 7C: Tool marker
- 7D: Rotational angle detection marker
- 8: Marker detecting unit
- 9: Navigation and maneuvering computer
- 10: Actuator main body
- 13: Spindle
- 22: Rotary shaft
- 31: Attitude altering member
- 41: Tool rotation drive source
- 42: Attitude altering drive source
- 45: Tool attitude detector
- 50: Operator unit
- 52: Display unit
- 54: Tool and tool marker relative position storage section
- 55: Tool and tool marker relative position and attitude storage section
- 56: Tool processing member position estimator
- 56a: Actuator display information generator
- 57: Object display information generator
- 58: Calibrating member
- 58a: Curved face
- 59: Actuator shape storage section
- Q1: Distal point
- Q2: Calibrating point

## Claims

1. A navigation system for estimating a position of a processing member of a tool relative to a remote controlled actuator, the remote controlled actuator comprising an actuator main body, in which a base end of a spindle guide section of an elongated configuration is connected with a drive unit housing; a distal end member fitted to a distal end portion of the spindle guide section for pivotal movement about a pivot center to enable it to be altered in attitude; the tool rotatably supported by the distal end member; an attitude altering drive source and a tool rotation drive source both provided within the drive unit housing for altering the attitude of the distal end member and rotating the tool, respectively; and an operator unit provided in the drive unit housing for controlling respective operations of the drive sources for maneuvering the attitude of the distal end member and the rotation of the tool, the navigation system comprising:
a marker detecting unit for detecting respective positions and attitudes of a main body marker, fitted to the drive unit housing of the actuator main body, and a tool marker provided at a predetermined relative position relative to the processing member of the tool;
a tool and tool marker relative position storage section for storing a relative position of the processing member of the tool relative to the tool marker;
a tool attitude detector for detecting an angle of rotation of the distal end member relative to the actuator main body about the pivot center;
a tool marker relative position and attitude storage section in which for each angle of rotation of the distal end member detected by the tool attitude detector, the relative position and attitude of the tool marker relative to the main body marker, which have been calculated from the position and attitude of the main body marker and the position and attitude of the tool marker both detected by the marker detecting unit, are recorded; and
a tool processing member position estimator for estimating the position of the processing member of the tool by estimating an absolute position and attitude of the tool marker from the position and attitude of the main body marker, detected by the marker detecting unit, and the relative position of the tool marker relative to the main body marker, which is obtained by checking the angle of rotation of the distal end member, detected by the tool attitude detector, with reference to the tool marker relative position and attitude storage section, and by checking a result of such estimation with stored information of the tool and tool marker relative position storage section to thereby estimate the position of the processing member of the tool.

2. The navigation system for the remote controlled actuator as claimed in claim 1, in which the tool marker is fitted to a calibrating member removably mounted on the distal end member.

3. The navigation system for the remote controlled actuator as claimed in claim 2, in which the processing member of the tool is of a curved configuration, at least a portion of an outer surface thereof, which contains a tip end point located on a rotational center line of the tool, being convexed outwardly thereof and in which the calibrating member has a calibrating point that is held in contact with the tip end point of the processing member, a portion of the calibrating member in proximate to the calibrating point having an outer surface of a curved shape concaved along a curved outer surface of the processing member.

4. The navigation system for the remote controlled actuator as claimed in claim 1, in which each of the main body marker and the tool marker is of a type capable of projecting or reflecting light and the marker detecting unit is of an optical type capable of receiving light from the main body marker and the tool marker.

5. The navigation system for the remote controlled actuator as claimed in claim 1, in which the tool attitude detector is provided in the attitude altering drive source or a drive system for transmitting an operation from the attitude altering drive source to the distal end member and is operable to output an electric signal corresponding to the attitude of the distal end member.

6. The navigation system for the remote controlled actuator as claimed in claim 1, further comprising a display unit for displaying images and in which the tool processing member position estimator is provided with an actuator display information generator for calculating an actuator display information, which is information for displaying the position and attitude of the actuator main body and the attitude of the distal end member, from various input information that is used in the estimation of the position of the tool, and then displaying such actuator display information on a screen of the display unit.

7. The navigation system for the remote controlled actuator as claimed in claim 6, in which the actuator display information generated by the actuator display information generator is information necessary to display the position and attitude of the actuator main body and the attitude of the distal end member on a screen of the display unit in the form of series of dots.

8. The navigation system for the remote controlled actuator as claimed in claim 6, in which the actuator display information generator is of a type generating, as the actuator display information, a graphic symbol and then displaying the graphic symbol on a screen of the display unit, which symbol is representative of an external shape of the tool, the distal end member and the actuator main body, which reflect the position and attitude of them, by means of a computer graphics.

9. The navigation system for the remote controlled actuator as claimed in claim 6, in which the actuator display information generated by the actuator display information generator is information in the form of a numeral on a screen of the display unit.

10. The navigation system for the remote controlled actuator as claimed in claim 1, in which the distal end member rotatably supports the spindle for holding the tool; the spindle guide section includes a rotatable shaft for transmitting rotation of the tool rotation drive source within the drive unit housing to the spindle and an attitude altering member for altering the attitude of the distal end member by selectively advancing or retracting by means of the attitude altering drive source within the drive unit housing in a condition with the tip end held in contact with the distal end member.
